# EUROPÄISCHE PATENTSCHRIFT

(11) **EP 2 041 276 B1**
(45) Veröffentlichungstag und Bekanntmachung des Hinweises auf die Patenterteilung: **29.02.2012**
(21) Anmeldenummer: 07786778.6
(22) Anmeldetag: 20.06.2007
(51) Int. Cl.: C12N 9/12

(54) **VERFAHREN ZUR HERSTELLUNG VON L-AMINOSÄUREN MITTELS MUTANTEN DES GLTA-GENS KODIEREND FÜR CITRATSYNTHASE**
METHOD FOR PRODUCING L-AMINO ACIDS BY MEANS OF MUTANTS OF THE GLTA GENE CODING FOR CITRATE SYNTHASE
PROCÉDÉ DE PRODUCTION DE L-AMINOACIDES AU MOYEN DU GÈNE GLTA CODANT CITRATE SYNTHASE

(30) Priorität: 13.07.2006 DE 102006032634
(43) Veröffentlichungstag der Anmeldung: 01.04.2009
(73) Patentinhaber: Evonik Degussa GmbH, 45128 Essen (DE)
(72) Erfinder: BATHE, Brigitte, 33154 Salzkotten (DE); CLAES, Wilfried, 33615 Bielefeld (DE)
(86) Internationale Anmeldenummer: PCT/EP2007/056153
(87) Internationale Veröffentlichungsnummer: WO 2008/006680

(56) Entgegenhaltungen:
- EP-A- 0 551 614
- GEORGI ET AL: "Lysine and glutamate production by Corynebacterium glutamicum on glucose, fructose and sucrose: Roles of malic enzyme and fructose-1,6-bisphosphatase" METABOLIC ENGINEERING, ACADEMIC PRESS,, US, Bd. 7, Nr. 4, Juli 2005 (2005-07), Seiten 291-301, XP005052640 ISSN: 1096-7176
- DATABASE BIOSIS [Online] BIOSCIENCES INFORMATION SERVICE, PHILADELPHIA, PA, US; 1988, YOKOTA A ET AL: "EFFECTS OF REDUCED CITRATE SYNTHASE ACTIVITY AND FEEDBACK-RESISTANT PHOSPHOENOLPYRUVATE CARBOXYLASE ON LYSINE PRODUCTIVITIES OF BREVIBACTERIUM-FLAVUM MUTANTS" XP002477332 Database accession no. PREV198886014353 & AGRICULTURAL AND BIOLOGICAL CHEMISTRY, Bd. 52, Nr. 2, 1988, Seiten 455-464, ISSN: 0002-1369

## Beschreibung

Gegenstand der Erfindung sind neue, für ein Polypeptid mit Citratsynthase Aktivität kodierende Polynukleotide, Bakterien, die diese enthalten, und Verfahren zur Herstellung von Aminosäuren unter Verwendung dieser Bakterien.

### Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen der Gattung Corynebacterium, insbesondere Corynebacterium glutamicum, eingesetzt, indem man einzelne Aminosäure-Biosynthesegene amplifiziert und die Auswirkung auf die Aminosäure-Produktion untersucht.

Zusammenfassende Darstellungen zur Biologie, Genetik und Biotechnologie von Corynebacterium glutamicum sind im "Handbook of Corynebacterium glutamicum" (Eds.: L. Eggeling und M. Bott, CRC Press, Taylor & Francis, 2005), in der Spezialausgabe des Journal of Biotechnolgy (Chief Editor: A. Pühler) mit dem Titel "A New Era in Corynebacterium glutamicum Biotechnology" (Journal of Biotechnolgy 104/1-3, (2003)) und im Buch von T. Scheper (Managing Editor) "Microbial Production of L-Amino Acids" (Advances in Biochemical Engineering/Biotechnology 79, Springer Verlag, Berlin, Deutschland, 2003) zu finden.

Die Nukleotidsequenz des Genoms von Corynebacterium glutamicum ist bei Ikeda und Nakagawa (Applied Microbiology and Biotechnology 62, 99-109 (2003)), in der EP 1 108 790 und bei Kalinowski et al. (Journal of Biotechnolgy 104/1-3, (2003)) beschrieben.

Die Nukleotidsequenz des Genoms von Corynebacterium efficiens ist bei Nishio et al (Genome Research. 13 (7), 1572-1579 (2003)) beschrieben.

Die Nukleotidsequenzen des Genoms von Corynebacterium glutamicum und Corynebacterium efficiens sind ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Die aus dem Stand der Technik bekannte Wildtypsequenz der Kodierregion des gltA-Gens von Corynebacterium glutamicum ist in der SEQ ID NO: 1 im Beschreibungsteil der vorliegenden Anmeldung dargestellt. In der SEQ ID NO: 3 und 25 sind darüberhinaus die stromaufwärts und stromabwärts der Kodierregion gelegenen Sequenzen dargestellt. Die Aminosäuresequenz des kodierten GltA-Polypeptids (Citratsynthase) ist dementsprechend in den SEQ ID NO's: 2, 4 und 26 wiedergegeben.

### Aufgabe der Erfindung

Die Erfinder haben sich zur Aufgabe gestellt neue Maßnahmen zur verbesserten Herstellung von L-Aminosäuren, bevorzugt L-Lysin, L-Valin und L-Isoleucin, besonders bevorzugt L-Lysin bereitzustellen.

### Beschreibung der Erfindung

Gegenstand der Erfindung ist ein isoliertes Polynukleotid, das für ein Polypeptid kodiert, welches die Aminosäuresequenz von SEQ ID NO:2 umfasst, wobei die L-Asparaginsäure an Position 5 der Aminosäuresequenz durch L-Valin ersetzt ist und wobei das Polypeptid Citratsynthase Aktivität (EC Nr. 4.1.3.7) besitzt. Gegebenenfalls enthält das Polypeptid einen oder mehrere konservative Aminosäureaustausch(e), wobei durch die konservativen Aminosäureaustausche die Citratsynthase-Aktivität des Polypeptids im wesentlichen unverändert bleibt.

Unter proteinogenen Aminosäuren versteht man die Aminosäuren, die in natürlichen Proteinen, das heißt in Proteinen von Mikroorganismen, Pflanzen, Tieren und Menschen vorkommen. Hierzu gehören insbesondere L-Aminosäuren, ausgewählt aus der Gruppe L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin.

Die Begriffe Polypeptid und Protein werden als Synonyme verwendet.

Gegenstand der Erfindung sind weiterhin Vektoren und Bakterien, bevorzugt der Gattung Corynebacterium und Escherichia, besonders bevorzugt der Spezies Corynebacterium glutamicum und Escherichia coli, die das genannte Polynukleotid enthalten oder unter Verwendung des genannten Polynukleotids hergestellt wurden.

Gegenstand der Erfindung sind schließlich Bakterien bevorzugt der Gattung Corynebacterium und Escherichia, besonders bevorzugt der Spezies Corynebacterium glutamicum und Escherichia coli, die mit dem genannten Vektor transformiert worden sind.

Der Begriff Transformation umfasst sämtliche Methoden zur Übertragung von Polynukleotiden, insbesondere DNA, in ein gewünschtes Bakterium. Hierzu gehören unter anderem die Verwendung von isolierter DNA bei der Transformation, Elektrotransformation bzw. Elektroporation, die Übertragung durch Zellkontakt wie bei der Konjugation oder die Übertragung von DNA mittels Partikelbeschuss.

Ein weiterer Aspekt der Erfindung betrifft ein Bakterium, insbesondere rekombinantes Bakterium, der Gattung Corynebacterium, welches ein Polynukleotid enthält, das für ein Polypeptid mit Citratsynthase-Aktivität kodiert, welches die Aminosäuresequenz von SEQ ID NO:2 umfasst, wobei an Position 5 der Aminosäuresequenz L-Valin, enthalten ist. Gegebenenfalls enthält das Polypeptid einen oder mehrere konservative Aminosäureaustausch(e), wobei durch die konservativen Aminosäureaustausche die Citratsynthase-Aktivität des Polypeptids im wesentlichen unverändert bleibt.

Ein weiterer Aspekt der Erfindung betrifft ein Verfahren zur Herstellung von L-Aminosäuren, bevorzugt L-Lysin, L-Valin und L-Isoleucin, besonders bevorzugt L-Lysin, dadurch gekennzeichnet, dass es folgende Schritte umfasst:
a) Fermentation der erfindungsgemäßen rekombinanten Bakterien der Gattung Corynebacterium in einem geeignetem Nährmedium, und
b) Akkumulation der L-Aminosäure in dem Nährmedium oder in den Zellen der Bakterien.

Mit L-Aminosäuren sind die proteinogenen Aminosäuren gemeint.

Wird im Folgenden L-Lysin oder Lysin erwähnt, sind damit nicht nur die Basen, sondern sind auch die Salze wie z.B. L-Lysin-Monohydrochlorid oder L-Lysin-Sulfat gemeint.

Bei den Bakterien der Gattung Corynebacterium werden L-Aminosäure-ausscheidende Stämme bevorzugt, die auf folgenden Arten beruhen:
Corynebacterium efficiens, wie zum Beispiel der Stamm DSM44549,
Corynebacterium glutamicum, wie zum Beispiel der Stamm ATCC13032,
Corynebacterium thermoaminogenes wie zum Beispiel der Stamm FERM BP-1539, und
Corynebacterium ammoniagenes, wie zum Beispiel der Stamm ATCC6871,
wobei die Art Corynebacterium glutamicum ganz besonders bevorzugt wird.

Einige Vertreter der Art Corynebacterium glutamicum sind im Stand der Technik auch unter anderen Bezeichnungen bekannt. Hierzu gehören beispielsweise:
Corynebacterium acetoacidophilum ATCC13870,
Corynebacterium lilium DSM20137,
Corynebacterium melassecola ATCC17965,
Brevibacterium flavum ATCC14067,
Brevibacterium lactofermentum ATCC13869, und
Brevibacterium divaricatum ATCC14020.

Bekannte Vertreter Aminosäure-ausscheidender Stämme der Gattung Corynebacterium sind beispielsweise die L-Lysin produzierenden Stämme:
Corynebacterium glutamicum DM58-1/pDM6 (= DSM4697) beschrieben in EP 0 358 940,
Corynebacterium glutamicum MH20-22B (= DSM16835) beschrieben in Menkel et al. (Applied and Environmental Microbiology 55(3), 684-688 (1989)),
Corynebacterium glutamicum AHP-3 (= Ferm BP-7382) beschrieben in EP 1 108 790,
Corynebacterium glutamicum DSM16834 beschrieben in (PCT/EP2005/012417),
Corynebacterium glutamicum DSM17119 beschrieben in (PCT/EP2006/060851),
Corynebacterium glutamicum DSM17223 beschrieben in (PCT/EP2006/062010),
Corynebacterium glutamicum DSM16937 beschrieben in (PCT/EP2005/057216), und
Corynebacterium thermoaminogenes AJ12521 (= FERM BP-3304) beschrieben in US 5,250,423;
oder die L-Valin produzierenden Stämme:
Brevibacterium lactofermentum FERM BP-1763 beschrieben in US 5,188,948,
Brevibacterium lactofermentum FERM BP-3007 beschrieben in US 5,521,074,
Corynebacterium glutamicum FERM BP-3006 beschrieben in US 5,521,074, und
Corynebacterium glutamicum FERM BP-1764 beschrieben in US 5,188,948;
oder die L-Isoleucin produzierenden Stämme:
Brevibacterium flavum FERM-BP 759
beschrieben in US 4,656,135,
Corynebacterium glutamicum FERM-BP 757.
beschrieben in US 4,656,135,
Brevibacterium flavum FERM-BP 760.
beschrieben in US 4,656,135,
Corynebacterium glutamicum FERM-BP 758
beschrieben in US 4,656,135,
Brevibacterium flavum FERM BP-2215
beschrieben in US 5,705, 370, und
Brevibacterium flavum FERM BP-2433
beschrieben in US 5,705, 370.

Angaben zur taxonomischen Einordnung von Stämmen dieser Gruppe von Bakterien findet man unter anderem bei Seiler (Journal of General Microbiology 129, 1433-1477 (1983)), Kämpfer und Kroppenstedt (Canadian Journal of Microbiology 42, 989-1005 (1996)), Liebl et al (International Journal of Systematic Bacteriology 41, 255-260 (1991)) und in der US 5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Der Stamm Corynebacterium thermoaminogenes (FERM BP-1539, ist in der US 5,250,434 beschrieben.

Zur Herstellung der erfindungsgemäßen Polynukleotide können klassische in-vivo Mutageneseverfahren mit Zellpopulationen von Bakterien der Gattung Corynebacterium unter Verwendung mutagener Stoffe wie beispielsweise N-Methyl-N'-Nitro-N-Nitrosoguanidin (MNNG) oder von ultraviolettem Licht verwendet werden. Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al (Folia Microbiologica 33, 337-343 (1988)) beschrieben.

Aus der mutagenisierten Zellpopulation werden diejenigen Mutanten entnommen und vermehrt, welche L-Glutaminsäure oder Zitronensäure benötigen, um auf einem Minimalagar wachsen zu können oder deren Wachstum auf Minimalagar durch Gabe von L-Glutaminsäure bzw. Zitronensäure verbessert wird. Es ist weiterhin möglich ausgehend von den L-Glutaminsäure- bzw. Zitronensäure-bedürftigen Mutanten sogenannte Revertanten zu isolieren, die für ihr Wachstum die L-Glutaminsäure bzw. Zitronensäure nicht benötigen. Diese L-Glutaminsäure- bzw. Zitronensäure-auxotrophen Mutanten beziehungsweise deren Revertanten werden anschließend untersucht. Technische Einzelheiten zur Isolierung von Mutanten mit defekter Citratsynthase Aktivität können beispielsweise bei Shiio et al. (Agricultural and Biological Chemistry 46(1), 101-107 (1982)) nachgelesen werden.

Anschließend wird aus den Mutanten DNA bereitgestellt, beziehungsweise isoliert und mit Hilfe der Polymerase-Kettenreaktion (PCR) unter Verwendung von Primerpaaren, die die Amplifizierung des gltA-Gens bzw. gltA-Allels erlauben, das entsprechende Polynukleotid synthetisiert und isoliert.

Hierzu können beliebige Primerpaare aus der stromaufwärts und stromabwärts der Kodierregion gelegenen Nukleotidsequenz und der dazu komplementären Nukleotidsequenz ausgewählt werden (Siehe SEQ ID NO's: 3 und 25). Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 1000 von SEQ ID NO:25. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz zwischen Position 3314 und 2312 von SEQ ID NO:25.

Anleitungen und Informationen zur PCR findet der Fachmann beispielsweise im Handbuch "PCR-Strategies" (Innis, Felfand und Sninsky, Academic Press, Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994).

Weitere Anleitungen zur PCR finden sich beispielsweise in der WO 06/100177 auf Seiten 15 bis 17.

In einem weiteren Arbeitschritt wird dann die Nukleotidsequenz des Polynukleotids bestimmt. Diese kann beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067pp (1990)) angegebenen Modifikationen bestimmt werden.

Das von dieser Nukleotidsequenz kodierte Polypeptid kann dann bezüglich der Aminosäuresequenz analysiert werden. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Es ist ebenfalls möglich das erfindungsgemäße Polynukleotid, das im Folgenden auch als erfindungsgemäßes gltA-Allel bezeichnet wird, durch Methoden der in vitro Genetik herzustellen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992) oder der Einsatz einer Polymerasekettenreaktion unter Verwendung einer DNA-Polymerase, die eine hohe Fehlerquote aufweist. Eine derartige DNA-Polymerase ist beispielsweise die Mütazyme DNA Polymerase (GeneMorph PCR Mutagenesis Kit, Nr.600550) der Firma Stratagene (LaJolla, CA, USA). Weiterhin können mutagene Oligonukleotide, wie bei T. A. Brown (Gentechnologie für Einsteiger, Spektrum Akademischer Verlag, Heidelberg, 1993) und R. M. Horton (PCR-Mediated Recombination and Mutagenesis, Molecular Biotechnology 3, 93-99 (1995)) beschrieben, eingesetzt werden. Die von Papworth et al. (Strategies 9(3), 3-4 (1996)) beschriebene Methode unter Verwendung des "Quik Change Site-directed Mutagenesis Kit" der Firma Stratagene (La Jolla, California, USA) kann ebenfalls eingesetzt werden.

Methoden zur Bestimmung der Citratsynthase Aktivität können bei Eikmanns et al. (Microbiology 140, 1817-1828 (1994)) und bei Shiio et al. (Agricultural and Biological Chemistry 46(1), 101-107 (1982)) nachgelesen werden.

Es ist weiterhin möglich, das erfindungsgemäße Allel der Citratsynthase in Corynebacterium glutamicum oder Escherichia coli zu überexprimieren, um es beispielsweise anschließend in aufgereinigter beziehungsweise isolierter Form darzustellen.

Auf diese Weise wurde ein Polynukleotid isoliert, das die in SEQ ID NO:5 dargestellte Nukleotidsequenz aufweist. Das von diesem Polynukleotid kodierte Polypeptid ist in SEQ ID NO:6 und 8 dargestellt. Es enthält an Position 5 der Aminosäuresequenz L-Valin anstelle L-Asparaginsäure.

Es wurde gefunden, dass der Stamm ATCC13032, wenn er anstelle des Wildtyp gltA-Gens mit dem erfindungsgemäßen gltA-Allel ausgestattet ist, welches für die Citratsynthase gemäß SEQ ID NO:6 kodiert (ATCC13032::gltA D5V), im Vergleich zum Wildtyp Stamm ATCC13032, welcher die Citratsynthase gemäß SEQ ID NO:2 enthält, eine um ca. 40% bis maximal ca. 90% verringerte Enzymaktivität, bevorzugt eine um ca. 70% bis maximal ca. 90% verringerte Enzymaktivität aufweist.

Es ist bekannt, dass konservative Aminosäureaustausche die Enzymaktivität nur unwesentlich verändern. Gegenstand der Erfindung sind dementsprechend auch Polynukleotide, die für Polypeptide mit Citratsynthase Aktivität kodieren, welche zusätzlich zu den erfindungsgemäßen Aminosäureaustauschen an Position 5 der Aminosäuresequenz einen (1) oder mehrere konservative(n) Aminosäureaustausch(e) enthalten, welche(r) die enzymatische Aktivität im wesentlichen nicht ändert. Das heisst sie bleibt im wesentlichen unverändert. Der Begriff "im wesentlichen nicht geändert" bzw. "im wesentlichen unverändert" bedeutet in diesem Zusammenhang, dass die Citratsynthase Aktivität des Polypeptids um maximal 20 %, bevorzugt maximal 10% und besonders bevorzugt um maximal 5% bis maximal 2% verändert ist im Vergleich zur Citratsynthase Aktivität des Polypeptids gemäß SEQ ID NO:10 oder SEQ ID NO:6, bevorzugt SEQ ID NO:6.

Für einen experimentellen Test wird das gltA-Gen des Stammes ATCC 13032 gegen das gltA-Allel, welches für ein Polypeptid, enthaltend den erfindungsgemäßen Aminosäureaustausch an Position 5 und einen oder mehrere konservative Aminosäureaustausch(e), kodiert, ausgetauscht. Anschließend wird der Stamm kultiviert, ein Zellextrakt hergestellt und die Citratsynthase-Aktivität bestimmt. Als Referenz wird die Citratsynthase-Aktivit in Stamm ATCC13032::gltA D5V bestimmt.

Anstelle von Stamm ATCC13032 können auch L-Lysin ausscheidende Stämme von Corynebacterium glutamicum eingesetzt werden, welche die Kodierregion des gltA-Gens des Wildtyps einschließlich der stromaufwärts gelegenen Nukleotidsequenzen, entsprechend der Nukleotidsequenz zwischen Position 1 und 2064 von SEQ ID NO:3, bevorzugt SEQ ID NO:3, enthalten. Geeignete Stämme sind beispielsweise DSM16833 beschrieben in PCT/EP2005/012417, DSM13994 beschrieben in EP 1 239 040 A2 oder DSM17576 beschrieben in DE 102005045301. In diese Stämme lässt sich durch Allelaustausch die entsprechende(n) Mutation(en) in die Kodierregion des gltA-Gens einführen.

Es ist ebenfalls möglich die Polypeptide zu reinigen und an den gereinigten Polypeptiden die Vergleichstests durchzuführen.

Das Enzym Citratsynthase (EC Nr. 4.1.3.7) katalysiert die Kondensationsreaktion von Oxalacetat und Acetyl-CoA, wobei als Reaktionsprodukte Zitronensäure und Coenzym A (CoA) entstehen. In der Kyoto Encyclopedia of Genes and Genomes (KEGG, Kanehisa Laboratory, Bioinformatics Center, Institute for Chemical Research, Kyoto University, Japan) wird dem Enzym die Nr. EC 2.3.3.1 zugeordnet.

Bei den aromatischen L-Aminosäuren spricht man von konservativen Austauschen, wenn L-Phenylalanin, L-Tryptophan und L-Tyrosin gegeneinander ausgetauscht werden. Bei den hydrophoben L-Aminosäuren spricht man von konservativen Austauschen, wenn L-Leucin, L-Isoleucin und L-Valin gegeneinander ausgetauscht werden. Bei den polaren L-Aminosäuren spricht man von konservativen Austauschen, wenn L-Glutamin und L-Asparagin gegeneinander ausgetauscht werden. Bei den basischen L-Aminosäuren spricht man von konservativen Austauschen, wenn L-Arginin, L-Lysin und L-Histidin gegeneinander ausgetauscht werden. Bei den sauren L-Aminosäuren spricht man von konservativen Austauschen, wenn L-Asparaginsäure und L-Glutaminsäure gegeneinander ausgetauscht werden. Bei den Hydroxyl-Gruppen enthaltenden L-Aminosäuren spricht man von konservativen Austauschen, wenn L-Serin und L-Threonin gegeneinander ausgetauscht werden.

Bevorzugt enthält das Polypeptid höchstens zwei (2), höchstens drei (3), höchstens vier (4) oder höchstens fünf (5) konservative Aminosäureaustausche zusätzlich zu dem erfindungsgemäßen Austausch an Position 5 von SEQ ID NO:2.

Es ist bekannt, dass durch wirtseigene Enzyme, sogenannte Aminopeptidasen, das endständige Methionin bei der Proteinsynthese entfernt wird.

Die isolierten Polynukleotide, die für die erfindungsgemäße Citratsynthase Variante kodieren, oder Teile davon, können dazu verwendet werden, um rekombinante Stämme der Gattung Corynebacterium, bevorzugt Corynebacterium glutamicum herzustellen, die den erfindungsgemäßen Aminosäureaustausch an Position 5 der Aminosäuresequenz des Citratsynthase-Polypeptids enthalten und die im Vergleich zum Ausgangsbeziehungsweise Elternstamm in verbesserter Weise L-Aminosäuren in das sie umgebende Medium abgeben oder im Zellinneren anhäufen.

Bevorzugt werden solche Ausgangsstämme verwendet, die bereits die Fähigkeit besitzen mindestens 1 g/l, bevorzugt mindestens 5 g/l, besonders bevorzugt mindestens 10 g/l der gewünschten L-Aminosäure in das sie umgebende Nährmedium auszuscheiden.

Eine verbreitete Methode zum Einbau von Mutationen in Gene von Bakterien der Gattung Corynebacterium, insbesondere der Art Corynebacterium glutamicum, ist die des Allelaustausches, die auch unter der Bezeichnung "gene replacement" bekannt ist. Bei diesem Verfahren wird ein DNA-Fragment, welches die interessierende Mutation enthält, in den gewünschten Stamm überführt und die Mutation durch wenigstens zwei Rekombinationsereignisse beziehungsweise "cross over"-Ereignisse in das Chromosom des gewünschten Stammes inkorporiert beziehungsweise die im betreffenden Stamm vorhandene Sequenz eines Gens gegen die mutierte Sequenz ausgetauscht.

Das DNA-Fragment, enthaltend die interessierende Mutation, liegt bei dieser Methode typischerweise in einem Vektor, insbesondere einem Plasmid, vor, das vorzugsweise von dem mit der Mutation zu versehenden Stamm nicht oder nur begrenzt, d. h. unter ausgewählten Kulturbedingungen, repliziert wird. Als Hilfs- oder Zwischenwirt, in dem der Vektor replizierbar ist, wird im Allgemeinen ein Bakterium der Gattung Escherichia, bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli aber nicht in Corynebacterium replikativ. Besonders geeignet sind Vektoren, die ein konditional negativ dominant wirkendes Gen wie beispielsweise das sacB-Gen (Levansucrase-Gen) von beispielsweise Bacillus oder das galK-Gen (Galaktosekinase-Gen) von beispielsweise Escherichia coli enthalten. Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat. Diese ermöglichen die Selektion auf Rekombinationsereignisse, bei denen der Vektor aus dem Chromosom eliminiert wird.

Weiterhin wurde von Nakamura et al. (US 6,303,383) ein Temperatur-sensitives Plasmid für Corynebacterium beschrieben, das lediglich bei Temperaturen unterhalb von 31°C replizieren kann. Es kann ebenfalls für die Massnahmen der Erfindung eingesetzt werden.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das Corynebacterium überführt. Gegebenenfalls kann die Überführung der DNA auch durch ballistische Methoden (z. B. Partikelbeschuss) erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium. Als Zielgen bezeichnet man das Gen, in dem der gewünschte Austausch erfolgen soll.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Von Schwarzer und Pühler (Bio/Technology 9, 84-87 (1991) wurde diese Methode verwendet um ein lysA-Allel, welches eine Deletion trug, und um ein lysA-Allel, welches eine Insertion trug in das Chromosom von C. glutamicum anstelle des Wildtypgens einzubauen.

Von Nakagawa et al. (EP 1108790) und Ohnishi et al. (Applied Microbiology and Biotechnology 58(2), 217-223 (2002)) wurde diese Methode eingesetzt um verschiedene Mutationen ausgehend von den isolierten Allelen bzw. Polynukleotiden in das Chromosom von C. glutamicum einzubauen. Auf diese Weise gelang es Nakagawa et al. eine als Val59Ala bezeichnete Mutation in das Homoserin-Dehydrogenase Gen (hom), eine als Thr311Ile bezeichnete Mutation in das Aspartatkinase-Gen (lysC bzw. ask), eine als Pro458Ser bezeichnete Mutation in das Pyruvat-Carboxylase-Gen (pyc) und eine als Ala213Thr bezeichnete Mutation in das Glucose-6-phoshat-Dehydrogenase Gen (zwf) von C. glutamicum Stämmen einzubauen.

Für den Einbau der erfindungsgemäßen Mutation im gltA-Gen in das Chromosom mittels Allelaustausch kann ein Polynukleotid verwendet werden, das für eine Aminosäuresequenz kodiert, die den erfindungsgemäßen Aminosäureaustausch an Position 5 von SEQ ID NO:2, wie in SEQ ID NO:10 dargestellt, aufweist und stromaufwärts und stromabwärts davon eine Nukleotidsequenz mit einer Länge von jeweils mindestens ca. 51 (siehe auch SEQ ID NO:11 und 12) bevorzugt jeweils mindestens ca. 101 bzw. 102, (siehe auch SEQ ID NO:13 und 14) besonders bevorzugt jeweils mindestens ca. 201 Nukleobasen (siehe auch SEQ ID NO:15 und 16) und ganz besonders bevorzugt jeweils mindestens ca. 500 bzw. 498 Nukleobasen (siehe auch SEQ ID NO:17 und 18) ausgewählt aus SEQ ID NO:9 besitzt. Die maximale Länge der stromaufwärts und stromabwärts der Mutation gelegenen Nukleotidsequenz liegt im Allgemeinen bei ca. 500, ca. 750, ca. 1000, ca. 1500, ca. 2000 bis 2100 Nukleobasen. Die stromaufwärts der Mutation gelegene Nukleotidsequenz umfasst beispielsweise die Sequenz zwischen Position 1 bis 762 von SEQ ID NO:9 oder die Sequenz zwischen Position 1 bis 1012 von SEQ ID NO:25. Die stromabwärts der Mutation gelegene Nukleotidsequenz umfasst beispielsweise die Sequenz zwischen Position 766 bis 2814 von SEQ ID NO:9 oder die Sequenz zwischen Position 1016 bis 3314 von SEQ ID NO:25. Die Gesamtlänge des für den Allelaustausch eingesetzten Polynukleotids beträgt dementsprechend maximal ca. 1000, maximal ca. 1500, maximal ca. 2000, maximal ca. 3000 oder maximal ca. 4000 bis 4200 Nukleobasen.

Gegenstand der Erfindung ist dementsprechend ein Polynukleotid, das eine Nukleotidsequenz umfasst, welche von Position 1 bis 39 die Nukleotidsequenz entsprechend Position 1 bis 39 von SEQ ID NO:11 und von Position 40 bis 105 eine Nukleotidsequenz enthält, die für die Aminosäuresequenz gemäß SEQ ID NO:12 kodiert, wobei an Position 5 L-Valin enthalten ist.

In diesem Zusammenhang entsprechen die Positionsangaben 1 bis 39 von SEQ ID NO:11 den Positionsangaben 712 bis 750 von SEQ ID NO:9. Die Positionsangaben 40 bis 105 von SEQ ID NO:11 entsprechen den Positionsangaben 751 bis 816 von SEQ ID NO:9.

In einer bevorzugten Form umfasst das Polynukleotid die Nukleotidsequenz von SEQ ID NO:11, wobei das Kodon entsprechend Position 52 bis 54 für L-Valin kodiert.

In einer besonders bevorzugten Ausführungsform umfasst das Polynukleotid die Nukleotidsequenz von Position 712 bis 816 von SEQ ID NO:7.

Gegenstand der Erfindung ist dementsprechend weiterhin ein Polynukleotid das eine Nukleotidsequenz umfasst, welche von Position 1 bis 89 die Nukleotidsequenz entsprechend Position 1 bis 89 von SEQ ID NO:13 und von Position 90 bis 206 eine Nukleotidsequenz enthält, die für die Aminosäuresequenz gemäß SEQ ID NO:14 kodiert, wobei an Position 5 L-Valin enthalten ist.

In diesem Zusammenhang entsprechen die Positionsangaben 1 bis 89 von SEQ ID NO:13 den Positionsangaben 662 bis 750 von SEQ ID NO:9. Die Positionsangaben 90 bis 206 von SEQ ID NO:13 entsprechen den Positionsangaben 751 bis 867 von SEQ ID NO:9.

In einer bevorzugten Form umfasst das Polynukleotid die Nukleotidsequenz von SEQ ID NO:13, wobei das Kodon entsprechend Position 102 bis 104 L-Valin kodiert.

In einer besonders bevorzugten Ausführungsform umfasst das Polynukleotid die Nukleotidsequenz von Position 662 bis 867 von SEQ ID NO:7.

Gegenstand der Erfindung ist dementsprechend schließlich ein Polynukleotid das eine Nukleotidsequenz umfasst, welche von Position 1 bis 189 die Nukleotidsequenz entsprechend Position 1 bis 189 von SEQ ID NO:15 und von Position 190 bis 405 eine Nukleotidsequenz enthält, die für die Aminosäuresequenz gemäß SEQ ID NO:16 kodiert, wobei an Position 5 L-Valin enthalten ist.

In diesem Zusammenhang entsprechen die Positionsangaben 1 bis 189 von SEQ ID NO:15 den Positionsangaben 562 bis 750 von SEQ ID NO:9. Die Positionsangaben 190 bis 405 von SEQ ID NO:15 entsprechen den Positionsangaben 751 bis 966 von SEQ ID NO:9.

In einer bevorzugten Form umfasst das Polynukleotid die Nukleotidsequenz von SEQ ID NO:15, wobei das Kodon entsprechend Position 202 bis 204 L-Valin kodiert.

In einer besonders bevorzugten Ausführungsform umfasst das Polynukleotid die Nukleotidsequenz von Position 562 bis 966 von SEQ ID NO:7.

Gegenstand der Erfindung ist dementsprechend schließlich auch ein Polynukleotid das eine Nukleotidsequenz umfasst, welche von Position 1 bis 488 die Nukleotidsequenz entsprechend Position 1 bis 488 von SEQ ID NO:17 und von Position 489 bis 1001 eine Nukleotidsequenz enthält, die für die Aminosäuresequenz gemäß SEQ ID NO:18 kodiert, wobei an Position 5 L-Valin enthalten ist.

In diesem Zusammenhang entsprechen die Positionsangaben 1 bis 488 von SEQ ID NO:17 den Positionsangaben 263 bis 750 von SEQ ID NO:9. Die Positionsangaben 489 bis 1001 von SEQ ID NO:15 entsprechen den Positionsangaben 751 bis 1263 von SEQ ID NO:9.

In einer bevorzugten Form umfasst das Polynukleotid die Nukleotidsequenz von SEQ ID NO:17, wobei das Kodon entsprechend Position 501 bis 503 für L-Valin kodiert.

In einer besonders bevorzugten Ausführungsform umfasst das Polynukleotid die Nukleotidsequenz von Position 263 bis 1263 von SEQ ID NO:7.

In einer ganz besonders bevorzugten Ausführungsform umfasst das Polynukleotid die Nukleotidsequenz von Position 9 bis 1687 von SEQ ID NO:31.

Gegenstand der Erfindung sind ebenfalls Vektoren, bevorzugt Plasmide, die die genannten Polynukleotide enthalten.

Gegenstand der Erfindung sind ebenfalls Bakterien bevorzugt der Gattung Escherichia, besonders bevorzugt der Art Escherichia coli, und Corynebacterium, besonders bevorzugt der Art Corynebacterium glutamicum, die die genannten Vektoren enthalten.

Gegenstand der Erfindung sind ebenfalls Stämme der Gattung Corynebacterium, bevorzugt der Spezies Corynebacterium glutamicum, die unter Verwendung der erfindungsgemäßen Polynukleotide oder unter Verwendung von Vektoren, die die erfindungsgemäßen Polynukleotide enthalten, hergestellt wurden.

Es ist ebenfalls möglich das erfindungsgemäße gltA-Allel an einen anderen Ort im Chromosom von Corynebacterium glutamicum einzubauen. Hierbei kann es sich beispielsweise um die Orte bzw. Gene aecD, ccpA1 ccpA2, citA, citB, citE, fda, gluA, gluB, gluC, gluD, luxR, luxS, lysR1, lysR2, lysR3, menE, mqo, pck, pgi und poxB handeln, so wie in der WO 03/04037 beschrieben. Hierbei kann es sich weiterhin beispielsweise um intergenische Regionen, DNA von Prophagen, defekten Phagen und Phagen Komponenten handeln, so wie in der WO 04/069996 beschrieben.

Die erfindungsgemäß erhaltenen, rekombinanten Stämme zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Die für die Massnahmen der Erfindung einsetzbaren L-Lysin ausscheidenden Ausgangstämme besitzen neben anderen Eigenschaften insbesondere eine Lysin-insensitive Aspartatkinase.

Unter einer Lysin-insensitive Aspartatkinase versteht man ein Polypeptid bzw. Protein mit Aspartatkinase-Aktivität (EC Nr 2.7.2.4), die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Derartige Aspartatkinasen werden auch als "feed back" resistente beziehungsweise desensibilisierte Aspartatkinasen bezeichnet. Die für diese desensibilisierten Aspartatkinasen bzw. Aspartatkinasevarianten kodierenden Nukleotidsequenzen werden auch als lysC^{FBR}-Allele bezeichnet. Informationen zu zahlreichen lysC^{FBR}-Allelen sind in den öffentlichen Datenbanken verfügbar. Einige Autoren bezeichnen das lysC-Gen auch als ask-Gen.

Die Kodierregion des Wildtyp lysC-Gens von Corynebacterium glutamicum entsprechend der Zugangsnummer AX756575 der NCBI Datenbank ist in SEQ ID NO:19 und das von diesem Gen kodierte Polypeptid in SEQ ID NO:20 dargestellt. In der SEQ ID NO:21 sind außerdem die stromaufwärts des 5'-Endes und stromabwärts des 3'-Endes der Kodierregion gelegenen Nukleotidsequenzen angegeben. SEQ ID NO:20 entspricht SEQ ID NO:22.

Die für die Massnahmen der Erfindung eingesetzten L-Lysin ausscheidenden Bakterien verfügen bevorzugt über ein lysC-Allel, das für eine Aspartatkinasevariante kodiert, welche die Aminosäuresequenz von SEQ ID NO:20 besitzt, wobei diese eine oder mehrere der Aminosäureaustausche ausgewählt aus der Gruppe:
a) LysC A279T (Austausch von L-Alanin an Position 279 des kodierten Aspartatkinaseproteins gemäß SEQ ID NO: 20 gegen L-Threonin; siehe US 5,688,671 und Zugangsnummern E06825, E06826, E08178 und 174588 bis 174597),
b) LysC A279V (Austausch von L-Alanin an Position 279 des kodierten Aspartatkinaseproteins gemäß SEQ ID NO: 20 gegen L-Valin, siehe JP 6-261766 und Zugangsnummer E08179),
c) LysC L297Q (Austausch von L-Leucin an Position 297 des kodierten Aspartatkinaseproteins gemäß SEQ ID NO: 20 gegen eine andere proteinogene Aminosäure, bevorzugt L-Glutamin; siehe DE 102006026328),
d) LysC S301F (Austausch von L-Serin an Position 301 des kodierten Aspartatkinaseproteins gemäß SEQ ID NO: 20 gegen L-Phenylalanin; siehe US 6,844,176 und Zugangsnummer E08180),
e) LysC S301Y (Austausch von L-Serin an Position 301 des kodierten Aspartatkinaseproteins gemäß SEQ ID NO: 20 gegen L-Tyrosin, siehe Kalinowski et al. (Molecular and General Genetics 224, 317-324 (1990)) und Zugangsnummer X57226),
f) LysC T308I (Austausch von L-Threonin an Position 308 des kodierten Aspartatkinaseproteins gemäß SEQ ID NO: 20 gegen L-Isoleucin; siehe JP 6-261766 und Zugangsnummer E08181)
g) LysC T311I (Austausch von L-Threonin an Position 311 des kodierten Aspartatkinaseproteins gemäß SEQ ID NO: 20 gegen L-Isoleucin; siehe WO 00/63388 und US 6,893,848),
h) LysC S317A (Austausch von L-Serin an Position 317 des kodierten Aspartatkinaseproteins gemäß SEQ ID NO: 20 gegen L-Alanin; siehe US 5,688,671 und Zugangsnummer 174589),
i) LysC R320G (Austausch von L-Arginin an Position 320 des kodierten Aspartatkinaseproteins gemäß SEQ ID NO: 20 gegen Glycin; siehe Jetten et al. (Applied Microbiology and Biotechnology 43, 76-82 (995)) und Zugangsnummer L27125),
j) LysC G345D (Austausch von Glycin an Position 345 des kodierten Aspartatkinaseproteins gemäß SEQ ID NO: 20 gegen L-Asparaginsäure; siehe Jetten et al. (Applied Microbiology and Biotechnology 43, 76-82 (995)) und Zugangsnummer L16848),
k) LysC T380I (Austausch von L-Threonin an Position 380 des kodierten Aspartatkinaseproteins gemäß SEQ ID NO: 20 gegen L-Isoleucin; siehe WO 01/49854 und Zugangsnummer AX192358), und
l) LysC S381F (Austausch von L-Serin an Position 381 des kodierten Aspartatkinaseproteins gemäß SEQ ID NO: 20 gegen L-Phenylalanin; siehe EP 0435132)
umfasst.

Ganz besonders bevorzugt werden Stämme, deren Aspartatkinasevarianten den Aminosäureaustausch LysC T311I oder einen oder mehrere der Aminosäureaustausche ausgewählt aus der Gruppe LysC A279T, LysC L297Q, LysC S317A, LysC T380I und LysC S381F enthalten.

Es ist naturgemäß ebenfalls möglich zunächst den Einbau der erfindungsgemäßen Mutation im gltA-Gen des Chromosoms eines Bakteriums der Gattung Corynebacterium durchzuführen und anschließend eine oder mehrere der gewünschte(n) Mutation(n) im lysC-Gen des betreffenden Stammes einzubauen.

In einer weiteren Ausführungsform liegen die beschriebenen Aspartatkinasen in dem Corynebacterium, welches den erfindungsgemäßen Aminosäureaustausch im gltA-Gen enthält, überexprimiert vor.

Unter Überexpression versteht man allgemein eine Erhöhung der intrazellulären Konzentration oder Aktivität einer Ribonukleinsäure, eines Proteins oder eines Enzyms im Vergleich zum Ausgangsstamm (Elternstamm) oder Wildtypstamm. Unter einem Ausgangsstamm (Elternstamm) versteht man den Stamm, an dem die zur Überexpression führende Massnahme durchgeführt wurde.

Die Erhöhung der Konzentration oder Aktivität lässt sich beispielsweise dadurch erzielen, dass man die Kopienzahl der entsprechenden Polynukleotide chromosomal oder extrachromosomal um mindestens eine Kopie erhöht.

Eine weit verbreitete Methode zur Erhöhung der Kopienzahl besteht darin, dass man das entsprechende Polynukleotid in einen Vektor, bevorzugt ein Plasmid, einbaut, der von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al. (Journal of Biotechnology 104, 27-40 (2003)).

Weiterhin kann man als Vektoren Transposons, Insertionselemente (IS-Elemente) oder Phagen einsetzen. Derartige genetische Systeme sind beispielsweise in den Patentschriften US 4,822,738, US 5,804,414 und US 5,804414. In gleicher Weise kann das in der WO 92/02627 beschriebene IS-Element ISaB1 oder das Transposon Tn 45 des Plasmids pXZ10142 (zitiert im "Handbook of Corynebacterium glutamicum" (Herausgeber: L. Eggeling und M. Bott)) verwendet werden.

Eine andere verbreitete Methode zur Erzielung einer Überexpression ist das Verfahren der chromosomalen Genamplifikation. Bei dieser Methode wird mindestens eine zusätzliche Kopie des interessierenden Polynukleotids in das Chromosom eines coryneformen Bakteriums eingefügt.

Derartige Amplifikationsverfahren sind beispielsweise in der WO 03/014330 oder WO 03/040373 beschrieben.

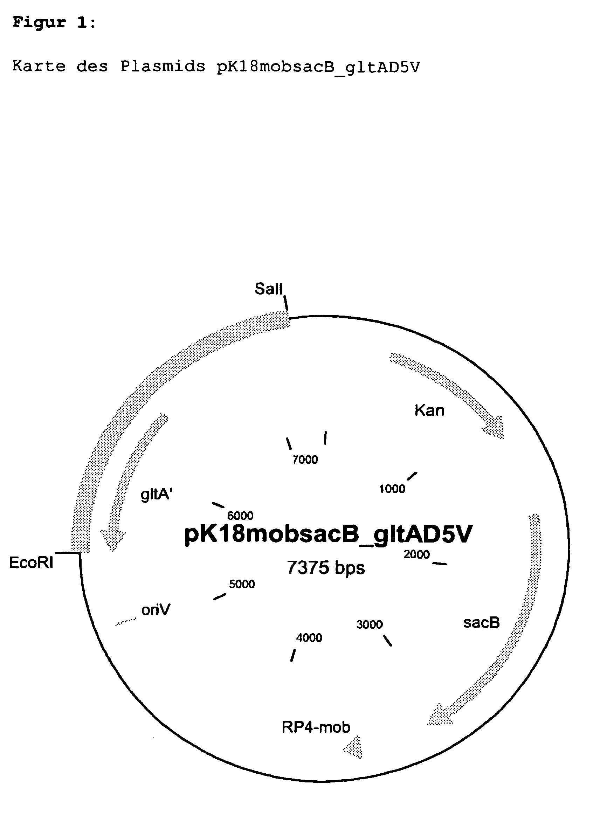

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in der Fig. 1 des Übersichtsartikel von Patek et al. (Journal of Biotechnology 104(1-3), 311-323 (2003)) beschrieben. In gleicher Weise können die von Vasicova et al (Journal of Bacteriology 181, 6188-6191 (1999)) beschrieben Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des betreffenden Gens, typischerweise im Abstand von ungefähr 1 - 500 Nukleobasen vom Startkodon eingefügt werden.

Durch die Maßnahmen der Überexpression, wird die Aktivität oder Konzentration des entsprechenden Polypeptids im allgemeinen um mindestens 10%, 25%, 50%, 75%, 100%, 150%, 200%, 300%, 400% oder 500%, maximal bis 1000% oder 2000% bezogen auf die Aktivität oder Konzentration des Polypeptids im Stamm vor der zur Überexpression führenden Massnahme, erhöht.

In einer weiteren Ausführungsform besitzen die erfindungsgemäßen Bakterien der Gattung Corynebacterium, die bevorzugt außerdem ein Polynukleotid enthalten, das für eine Lysin-insensitive Aspartatkinasevariante kodiert, eines oder mehrere der Merkmale ausgewählt aus der Gruppe
a) überexprimiertes Polynukleotid (dapA-Gen), das für eine Dihydrodipicolinat-Synthase (DapA, EC Nr. 4.2.1.52) kodiert,
b) überexprimiertes Polynukleotid (asd-Gen), das für eine Aspartatsemialdehyd-Dehydrogenase (Asd, EC Nr. 1.2.1.11) kodiert,
c) überexprimiertes Polynukleotid, (lysA-Gen), das für eine Diaminopimelat-Decarboxylase (LysA, EC Nr. 4.1.1.20) kodiert,
d) überexprimiertes Polynukleotid (aat-Gen), das für eine Aspartat-Aminotransferase (Aat, EC Nr. 2.6.1.1) kodiert,
e) überexprimiertes Polynukleotid (lysE-Gen), das für eine Polypeptid mit L-Lysin-Export Aktivität (LysE, Lysin Efflux Permease) kodiert,
f) ausgeschaltete oder abgeschwächte Aktivität der Malat-Dehydrogenase (Mdh, EC Nr. 1.1.1.37),
g) ausgeschaltete oder abgeschwächte Aktivität der Malat-Chinon Oxidoreduktase (Mqo, EC Nr. 1.1.99.16),
h) überexprimiertes Polynukleotid, das für eine Pyruvat-Carboxylase (Pyc, EC Nr. 6.4.1.1) kodiert, und
i) ausgeschaltete oder abgeschwächte Aktivität der E1p Untereinheit des Pyruvat-Dehydrogenase Komplexes (AceE, EC Nr. 1.2.4.1).

Die Merkmale a) bis g) werden besonders bevorzugt.

Zur Überexpression der aufgeführten Gene beziehungsweise Polynukleotide können die im Stand der Technik bekannten Gene, beispielsweise die sogenannten Wildtypgene, von Escherichia coli (Blattner et al., Science 277(5), 1453-1462 (1997)), Bacillus subtilis (Kunst et al, Nature 390 (6657), 249-256 (1997)), Bacillus licheniformis (Veith et al, Journal of Molecular Microbiology and Biotechnology 7 (4), 204-211 (2004)), Mycobacterium tuberculosis (Fleischmann et al, Journal of Bacteriology 1841, 5479-5490 (2004)), Mycobacterium bovis (Garnier et al, Proceedings of the National Academy of Sciences U.S.A. 100 (13), 7877-7882 (2003)), Streptomyces coeliclor (Redenbach et al, Molecular Microbiology 21 (1), 77-96 (1996)), Lactobacillus acidophilus (Altermann et al, Proceedings of the National Acadamy of Sciences U.S.A. 102 (11), 3906-3912 (2005)), Lactobacillus johnsonii (Pridmore et al, Proceedings of National Academy of Sciences U.S.A. 101 (8), 2512-2517 (2004)), Bifidobacterium longum (Schell et al, Proceedings of National Academy of Sciences U.S.A. 99 (22), 14422-14427 (2002)), und Saccharomyces cerevisiae verwendet werden. Die Genome der Wildtypformen dieser Bakterien liegen in sequenzierter und annotierter Form vor. Vorzugsweise werden die endogenen Gene beziehungsweise Polynukleotide der Gattung Corynebacterium, besonders bevorzugt der Art Corynebacterium glutamicum verwendet.

Unter endogenen Genen beziehungsweise Polynukleotiden versteht man die in der Population einer Art vorhandenen offenen Leserahmen (ORF), Gene oder Allele beziehungsweise deren Polynukleotide.

Das dapA-Gen von Corynebacterium glutamicum Stamm ATCC13032 ist beispielsweise in der EP 0 197 335 beschrieben. Zur Überexpression des dapA-Gens von Corynebacterium glutamicum können außerdem unter anderem die Mutationen MC20 und MA16 des dapA-Promotors, wie in der US 6,861,246 beschrieben, eingesetzt werden.

Das asd-Gen von Corynebacterium glutamicum Stamm ATCC 21529 ist beispielsweise in der US 6,927,046 beschrieben.

Das lysA-Gen von Corynebacterium glutamicum ATCC13869 (Brevibacterium lactofermentum) ist beispielsweise in der US 6,090,597 beschrieben.

Das aat-Gen von Corynebacterium glutamicum ATCC13032 ist beispielsweise bei Kalinowski et al (Journal of Biotechnology 104 (1-3), 5-25 (2003); siehe auch Zugangsnummer NC_006958) beschrieben. Es wird dort als aspB-Gen bezeichnet. In der US 6,004,773 wird ein für eine Aspartat-Aminotransferase kodierendes Gen als aspC bezeichnet. Marienhagen et al (Journal of Bacteriology 187 (22), 7639-7646 (2005) bezeichnen das aat-Gen als aspT-Gen.

Das lysE-Gen von Corynebacterium glutamicum R127 ist beispielweise in der US 6,858,406 beschrieben. Bei Stamm R127 handelt es sich um eine restriktionsdefekte Mutante von ATCC13032 (Liebl et al, FEMS Microbiology Letters 65, 299-304 (1989)). In gleicher Weise kann das in der US 6,861,246 verwendete lysE-Gen von Stamm ATCC13032 eingesetzt werden.

Das pyc-Gen von Corynebacterium glutamicum von Stamm ATCC 13032 ist beispielsweise in der WO 99/18228 und WO 00/39305 beschrieben. Weiterhin können Allele des pyc-Gens verwendet werden wie sie beispielsweise in der US 6,965,021 beschrieben sind. Die in dieser Patentschrift beschriebenen Pyruvat-Carboxylasen besitzen einen oder mehrere der Aminosäureaustausche ausgewählt aus der Gruppe: Pyc E153D (Austausch von L-Glutaminsäure an Position 153 gegen L-Asparaginsäure), Pyc A182S (Austausch von L-Alanin an Position 182 gegen L-Serin), Pyc A206S (Austausch von L-Alanin an Position 206 gegen L-Serin), Pyc H227R (Austausch von L-Histidin an Position 227 gegen L-Arginin), Pyc A455G (Austausch von L-Alanin an Position 455 gegen Glycin), und Pyc D1120E (Austausch von L-Asparaginsäure an Position 1120 gegen L-Glutaminsäure). In gleicher weise kann das in der EP 1 108 790 beschrieben pyc-Allel verwendet werden, das für eine Pyruvat-Carboxylase kodiert, welche den Aminosäureaustausch Pyc P458S (Austausch von L-Prolin an Position 458 gegen L-Serin) enthält.

Unter ausgeschalteter oder abgeschwächter Aktivität versteht man die Verringerung oder Ausschaltung der intrazellulären Aktivität oder Konzentration eines oder mehrerer Enzyme bzw. Proteine in einem Mikroorganismus, die durch das entsprechende Polynukleotid beziehungsweise DNA kodiert werden.

Zur Erzeugung eines Stammes, in dem die intrazelluläre Aktivität eines gewünschten Polypeptids ausgeschaltet ist, wird eine Deletion oder Insertion von mindestens einer (1) Nukleobase, bevorzugt von einer (1) oder von zwei (2) Nukleobasen, in die Kodierregion des entsprechenden Gens, eingebaut. Es ist gleichfalls möglich mindestens ein (1) oder auch mehrere Kodon(e) innerhalb der Kodierregion zu deletieren. Diese Massnahmen führen zu einer Verschiebung des Leserasters ("frame shift mutations") und damit typischerweise zur Synthese eines funktionsunfähigen Polypeptids. In gleicher Weise wirkt die Einführung einer Nichtsinnmutation ("nonsense mutation") durch Transversion oder Transition von mindestens einer (1) Nukleobase innerhalb der Kodierregion. Aufgrund des entstandenen Stop-Kodons kommt es zu einem vorzeitigem Abbruch der Translation. Die genannten Massnahmen werden bevorzugt in der Region zwischen dem Startkodon und dem vorletzten kodierenden Kodon, besonders bevorzugt im 5'-terminalen Teil der Kodierregion, durchgeführt, welcher für den N-Terminus des Polypeptids kodiert. Bezeichnet man die Gesamtlänge eines Polypeptids (gemessen als Anzahl der chemisch verbundenen L-Aminosäuren) mit 100%, so gehört - im Rahmen der vorliegenden Erfindung - zum N-Terminus des Polypeptids der Teil der Aminosäuresequenz, welcher, gerechnet ab der Start-Aminosäure L-Formyl-Methionin 80% der nachfolgenden L-Aminosäuren enthält.

Genetische Massnahmen zur Ausschaltung der Malat-Chinon Oxidoreduktase (Mqo) sind beispielsweise in der US 7,094,106 beschrieben.

Genetische Massnahmen zur Ausschaltung der Malat-Dehydrogenase (Mdh) sind beispielsweise in der WO 02/02778 beschrieben.

Genetische Massnahmen zur Ausschaltung der E1p Untereinheit (AceE) des Pyruvat-Dehydrogenase Komplexes sind beispielsweise in der EP 06119615 und bei Schreiner et al. (Journal of Bacteriology 187(17), 6005-6018 (2005)) beschrieben.

Es ist gleichfalls möglich durch geeignete Aminosäureaustausche die katalytische Eigenschaft des betreffenden Polypeptides zu senken.

Im Falle der Malat-Chinon Oxidoreduktase (Mqo) kann dies, wie in der WO 06/077004 beschrieben, dadurch erzielt werden, dass man Allele des mqo-Gens herstellt bzw. verwendet, welche für eine Mqo-Variante kodieren, die die Aminosäuresequenz von SEQ ID NO:24 besitzt und einen oder mehrere Aminosäureaustausche ausgewählt aus der Gruppe
a) Austausch des L-Serins an Position 111 gegen eine andere proteinogene Aminosäure, bevorzugt L-Phenylalanin oder L-Alanin, und
b) Austausch des L-Alanin an Position 201 gegen eine andere proteinogene Aminosäure, bevorzugt L-Serin,
enthält.

Ganz besonders bevorzugt werden Stämme die ein mqo-Allel enthalten, das für eine Mqo-Variante kodiert, welche die Aminosäuresequenz von SEQ ID NO:24 besitzt, wobei an Position 111 L-Phenylalanin enthalten ist.

Durch die Maßnahmen der Ausschaltung oder Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangsstamm bzw. Elternstamm herabgesenkt.

Die Leistung der erfindungsgemäß hergestellten Bakterien der Gattung Corynebacterium bzw. des Fermentationsprozesses unter Verwendung derselben bezüglich eines oder mehrerer der Parameter ausgewählt aus der Gruppe der L-Aminosäure-Konzentration (gebildete L-Aminosäure pro Volumen), der L-Aminosäure-Ausbeute (gebildete L-Aminosäure pro verbrauchter Kohlenstoff-Quelle), der L-Aminosäure-Bildung (gebildete L-Aminosäure pro Volumen und Zeit) und der spezifischen L-Aminosäure-Bildung (gebildete L-Aminosäure pro Zelltrockenmasse bzw. Biotrockenmasse und Zeit oder gebildete L-Aminosäure pro Zellprotein und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben erhöht.

Die erfindungsgemäß hergestellten Bakterien der Gattung Corynebacterium können kontinuierlich - wie beispielsweise in der PCT/EP2004/008882 beschrieben- oder diskontinuierlich im batch - Verfahren (Satzkultivierung bzw. Satzverfahren) oder im fed batch (Zulaufverfahren) oder repeated fed batch Verfahren (repetitives Zulaufverfahren) zum Zwecke der Produktion der gewünschten L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Lactose, Fructose, Maltose, Melasse, Saccharose-haltige Lösungen aus der Zuckerrüben-oder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten L-Aminosäure, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Bakterien kommt es zu einer Anreicherung (Akkumulation) der L-Aminosäure im Fermentationsmedium und/oder in den Bakterienzellen.

Beispiele für geeignete Fermentationsmedien finden sich unter anderem in den Patentschriften 5,770,409, US 5,840,551 und US 5,990,350 oder US 5,275,940.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Gegenstand der Erfindung ist demzufolge auch ein Verfahren zur Herstellung einer L-Aminosäure, dadurch gekennzeichnet, dass man folgende Schritte durchführt:
a) Fermentation der erfindungsgemäßen Bakterien in einem geeignetem Nährmedium,
b) Akkumulation der L- Aminosäure in dem Nährmedium oder in den Zellen der genannten Bakterien.

Daran schließt sich im Allgemeinen das Sammeln (collecting) der im Nährmedium bzw. in der Fermentationsbrühe oder in den Zellen der Bakterien akkumulierten L-Aminosäure an, um zu einem festen oder flüssigen Produkt zu gelangen.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten Aminosäure sicherstellen.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete gewünschte L-Aminosäure wie L-Lysin, L-Valin oder L-Isoleucin c) die im Laufe der Fermentation gebildeten organischen Nebenprodukte und d) die durch die Fermentation nicht verbrauchten Bestandteile des eingesetzten Fermentationsmediums beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten organischchemischen Verbindung erzeugt und gegebenenfalls ausgeschieden werden. Hierzu gehören auch Zucker wie zum Beispiel Trehalose.

Im Falle der Aminosäuren L-Valin und L-Isoleucin wird eine Isolierung und Reinigung, beispielsweise unter Verwendung einer oder mehrerer Verfahren ausgewählt aus der Gruppe chromatographische Verfahren, Kristallisationsverfahren und Verwendung von Aktivkohle, bevorzugt, so dass weitgehend reine Produkte, beispielsweise solche mit einer Reinheit von ≥90 Gew.-% oder ≥95 Gew.-% erhalten werden.

Im Falle der Aminosäure L-Lysin sind im Stand der Technik im wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen das L-Lysin aus der Fermentationsbrühe gesammelt, isoliert oder gereinigt wird, um das L-Lysin-haltige Produkt oder das gereinigte L-Lysin herzustellen.

Zur Herstellung von festem, reinen L-Lysin werden im wesentlichen Methoden der Ionenaustauschchromatographie gegebenfalls unter Verwendung von Aktivkohle und Methoden der Kristallisierung angewendet. Auf diese Weise erhält man die entsprechende Base oder ein entsprechendes Salz wie beispielsweise das Monohydrochlorid (Lys-HCl) oder das Lysinsulfat (Lys₂-H₂SO₄).

In der EP-B-0534865 ein Verfahren zur Herstellung wässriger, basischer L-Lysin-haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig, das heißt > 95% oder > 98% oder größer 99%, im Produkt. Enthält ein Produkt in diesem Sinn mindestens einen Teil der Bestandteile der Fermentationsbrühe, so wird dies auch mit dem Begriff "Produkt auf Fermentationsbrühebasis" umschrieben.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerfähiges und weitgehend staubfreies Produkt überführt werden.

Der Begriff "staubfrei" bedeutet, daß das Produkt lediglich geringe Anteile (< 5 %) an Körngrößen unter 100 µm Durchmesser enthält.

"Lagerfähig", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann, ohne dass ein wesentlicher Verlust (maximal 5%) der jeweiligen Aminosäure auftritt.

Ein weiterer Gegenstand der Erfindung ist ein Verfahren, das in seinen Grundzügen in der DE 102006016158 beschrieben ist, und bei dem die unter Verwendung der erfindungsgemäßen Mikroorganismen erhaltene Fermentationsbrühe, aus der die Biomasse gegebenenfalls ganz oder teilweise abgetrennt wurde, weiterverarbeitet wird, indem man ein Verfahren durchführt das mindestens folgende Schritte umfasst:
a) den pH-Wert durch Zugabe von Schwefelsäure auf 4,0 bis 5,2, insbesondere 4, 9 bis 5,1, absenkt, und ein molares Sulfat/L-Lysin-Verhältnis von 0,85 bis 1,2, bevorzugt 0,9 bis 1,0, besonders bevorzugt >0,9 bis <0, 95, in der Brühe einstellt, gegebenenfalls durch Zugabe von einer weiteren oder mehreren Sulfathaltigen Verbindung (en) und
b) das so erhaltene Gemisch durch Wasserentzug aufkonzentriert, und gegebenenfalls granuliert,
wobei gegebenenfalls vor Schritt a) eine oder beide der folgenden Massnahmen durchgeführt wird/werden:
c) Messung des molaren Verhältnisses von Sulfat/L-Lysin zur Ermittlung der benötigten Menge an Sulfat-haltigen Verbindung(n)
d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfit, besonders bevorzugt Natriumhydrogensulfit in einer Konzentration von 0,01 bis 0,5 Gew.-%, bevorzugt 0,1 bis 0,3 Gew.-%, besonders bevorzugt 0,1 bis 0,2 Gew.-% bezogen auf die Fermentationsbrühe hinzugesetzt.

Als bevorzugte Sulfat-haltigen Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [SO₄²⁻] / [L-Lysin] berechnet. Diese Formel berücksichtigt die Tatsache, dass das SO₄²⁻ Anion, bzw. die Schwefelsäure zweiwertig ist. Ein Verhältnis V = 1 bedeutet, dass ein stöchiometrisch zusammengesetztes Lys₂-H₂SO₄) vorliegt, während bei einem Verhältnis von V = 0,9 ein 10%iger Sulfatmangel und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu behandeln so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethylcellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken, Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten L-Lysin Konzentration im Produkt kann je nach Anforderung das L-Lysin während des Verfahrens in Form eines Konzentrates oder gegebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Ein weiterer Gegenstand der Erfindung ist ein Verfahren zur Herstellung eines festen Lysin-haltigen Produktes, wie es in seinen Grundzügen in der US 20050220933 beschrieben ist, und das die Aufarbeitung der unter Verwendung der erfindungsgemäßen Mikroorganismen erhaltenen Fermentationsbrühe in folgenden Schritten umfasst:
a) Filtration der Fermentationsbrühe, bevorzugt mit einem Membranfilter, so dass ein Biomasse-haltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s))

Zur Beschichtung in Schritt d) werden bevorzugt Beschichtungsmittel verwendet, welche ausgewählt werden aus der Gruppe, bestehend aus
d1) der in Schritt a) erhaltenen Biomasse,
d2) einer L-Lysin-haltigen Verbindung, bevorzugt ausgewählt aus der Gruppe L-Lysinhydrochlorid oder L-Lysinsulfat,
d3) einem im wesentlichen L-Lysin-freien Stoff mit L-Lysingehalt < 1 Gew.-%, bevorzugt < 0,5 Gew.-%, bevorzugt ausgewählt aus der Gruppe Stärke, Karageenan, Agar, Kieselsäuren, Silikate, Schrote, Kleien und Mehle, und
d4) einem wasserabstoßenden Stoff, bevorzugt ausgewählt aus der Gruppe Öle, Polyethylenglykole und flüssige Paraffine.

Durch die Massnahmen entsprechend den Schritten d1) bis d4), insbesondere d1) bis d3), wird der Gehalt an L-Lysin auf einen gewünschten Wert eingestellt.

Bei der Herstellung L-Lysin-haltiger Produkte wird das Verhältnis der Ionen bevorzugt so eingestellt, dass das molare Ionenverhältnis entsprechend nachstehender Formel 2x [SO₄²⁻] + [Cl⁻] - [NH₄⁺] - [Na⁺] - [K⁺] -2x [Mg²⁺] -2x [Ca²⁺] / [L-Lys] 0,68 bis 0,95, bevorzugt 0,68 bis 0,90 ergibt, so wie von Kushiki et al. in der US 20030152633 beschrieben.

Im Falle des Lysins hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Der Wassergehalt des L-Lysin-haltigen, festen Produktes beträgt bis zu 5 Gew.-%, bevorzugt bis zu 4 Gew.-%, und besonders bevorzugt weniger als 3 Gew.-%.

### Beispiel 1

### Sequenzierung des gltA-Gens des Stammes DM678

Der Stamm Corynebacterium glutamicum DM678 (US 6,861,246) ist ein Lysin-Produktionsstamm, der durch Mutagenese und Screening entwickelt wurde. Er ist auxotroph für L-Threonin und L-Methionin-sensitiv. Der Stamm wurde bei der Deutschen Sammlung für Mikroorganismen und Zellkulturen (DSMZ, Braunschweig, Deutschland) als DSM12866 hinterlegt.

Aus dem Stamm DM678 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher das gltA-Gen trägt. Hierfür wurden folgende Oligonukleotide als Primer verwendet:
gltA_XL-A1 (SEQ ID NO: 27):
   5' tgagttctattggcgtgacc 3'
gltA_XL-E1 (SEQ ID NO: 28):
   5' ttcgccaacgatgatgtcag 3'

Die dargestellten Primer wurden von der Firma MWG Biotech (Ebersberg, Deutschland) synthetisiert. Sie ermöglichen die Amplifizierung eines ca. 1,8 kb langen DNA-Abschnittes, welcher das gltA-Gen trägt. Der Primer gltA_XL-A1 bindet an die Region entsprechend Position 490 bis 509 des zu SEQ ID NO: 3 (und SEQ ID NO: 7) komplementären Stranges. Der Primer gltA_XL-E1 bindet an die Region ensprechend Position 2266 bis 2247 des Stranges gemäß SEQ ID NO: 3 (und SEQ ID NO: 7).

Die PCR-Reaktion wurde mit der Phusion High Fidelity DNA Polymerase (New England Biolabs, Frankfurt, Deutschland) durchgeführt. Der Reaktionsansatz wurde nach Herstellerangaben angesetzt und enthielt bei 50 µl GesamtVolumen 10 µl des mitgelieferten 5 x Phusion HF Buffer, Desoxynucleosidtriphosphate in einer Konzentration von jeweils 200 µM, Primer in einer Konzentration von 0,5 µM, ungefähr 50 ng Template-DNA und 2 Units Phusion Polymerase. Durch Zugabe von H₂O wurde das Volumen auf 50 µl eingestellt.

Der PCR-Ansatz wurde zuerst einer einleitenden Denaturierung bei 98°C für 30 Sekunden unterzogen. Darauf folgten sich 35x wiederholend ein Denaturierungsschritt bei 98°C für 20 Sekunden, ein Schritt zum Binden der Primer an die vorgelegte DNA bei 60°C für 20 Sekunden und der Extensionsschritt zur Verlängerung der Primer bei 72°C für 60 Sekunden. Nach dem abschliessenden Extensionsschritt für 5 Minuten bei 72°C wurde der PCR-Ansatz einer Agarosegel-Elektrophorese (0,8% Agarose) unterworfen. Ein DNA-Fragment von ca. 1,8 kb Länge wurde identifiziert, aus dem Gel isoliert und unter Verwendung des QIAquick Gel Extraction Kit der Firma Qiagen, (Hilden, Deutschland) aufgereinigt.

Die Nukleotidsequenz des amplifizierten DNA-Fragmentes bzw. PCR-Produktes wurde von der Firma Agowa (Berlin, Deutschland) bestimmt.

Die Nukleotidsequenz der Kodierregion des gltA-Allels aus dem Stamm DM678 enthält an Position 14 die Nukleobase Thymin. Das Gen des Wildtyps (siehe SEQ ID NO: 1) enthält an dieser Position die Nukleobase Adenin. Diese Adenin-Thymin Transversion führt zu einem Aminosäureaustausch von Aspartat zu Valin an Position 5 der resultierenden Aminosäuresequenz. Diese Mutation wird im Folgenden als gltAD5V bezeichnet. Das Allel gltAD5V ist in SEQ ID NO: 5 dargestellt, die sich mit Hilfe des Programmes Patentin ergebende Aminosäuresequenz des Proteins ist in der SEQ ID NO: 6 dargestellt.

### Beispiel 2

### Konstruktion des Austauschvektors pK18mobsacB_gltAD5V

Mit Hilfe der Polymerase-Kettenreaktion wurde ein DNA-Fragment amplifiziert, das einen Teil der "upstream-Region" des gltA-Gens sowie einen Teil der Kodierregion, welcher die Mutation gltAD5V enthält, trägt. Als Template wurde die in Beispiel 1 gewonnene chromosomale DNA von DM678 verwendet. Folgende Oligonukleotide wurden als Primer für die PCR ausgewählt:
gltA_1.p (SEQ ID NO: 29):
   5' CCGTCGACAATAGCCTGAA 3'
gltA_2.p (SEQ ID NO: 30):
   5' CC-GAATTC-TTCGAGCATCTCCAGAAC 3'

Sie wurden von der Firma MWG Biotech (Ebersberg, Deutschland) synthetisiert und ermöglichen die Amplifizierung eines ca. 1,7 kb langen DNA-Abschnittes, enthaltend 832 bp der upstream-Region und die Nukleotide 1-855 bp der Kodierregion des gltA-Gens aus DM678.

Der Primer gltA_1.p bindet an die Region entsprechend Position 169 bis 187 des zu SEQ ID NO: 25 komplementären Stranges. Die Nukleotide 9 bis 26 des Primers gltA_2.p binden an die Region ensprechend Position 1855 bis 1838 des Stranges gemäß SEQ ID NO: 25. Außerdem enthält der Primer gltA_1.p die native Schnittstelle des Restriktionsenzyms SalI und der Primer gltA_2.p die Sequenz der Schnittstelle des Restriktionsenzyms EcoRI, die in der oben dargestellten Nukleotidabfolge jeweils durch Unterstreichen markiert sind.

Die PCR-Reaktion wurde mit der Phusion High-Fidelity DNA Polymerase (New England Biolabs, Frankfurt, Deutschland) durchgeführt. Der Reaktionsansatz hatte die oben beschriebene Zusammensetzung. Die PCR wurde wie oben beschrieben durchgeführt. Die Nukleotidsequenz des ca. 1,7 kb langen Amplifikates ist in SEQ ID NO: 31 dargestellt.

Das Amplifikat wurde mit den Restriktionsendonukleasen SalI und EcoRI behandelt und durch Elektrophorese in einem 0,8%igen Agarosegel identifiziert. Es wurde anschließend aus dem Gel isoliert und mit dem QIAquick Gel Extraction Kit der Firma Qiagen aufgereinigt.

Das dergestalt gereinigte DNA-Fragment enthält die beschrieben gltAD5V-Mutation und besitzt kompatible Enden zu mit SalI bzw. EcoRI geschnittener DNA (gltAD5V-Fragment bzw. gltA in Figur 1). Es wurde anschließend in den von Schäfer et al. (Gene, 145, 69-73 (1994)) beschriebenen, mobilisierbaren Vektor pK18mobsacB kloniert, um einen Allel- beziehungsweise Mutationsaustausch zu ermöglichen. Hierzu wurde pK18mobsacB mit den Restriktionsenzymen EcoRI und SalI verdaut und die Enden mit alkalischer Phosphatase (Alkaline Phosphatase, Boehringer Mannheim, Deutschland) dephosphoryliert. Der so vorbereitete Vektor wurde mit dem gltAD5V-Fragment gemischt und der Ansatz mit dem Ready-To-Go T4 DNA Ligase Kit (Amersham-Pharmacia, Freiburg, Deutschland) behandelt.

Anschließend wurde der E.coli Stamm S17-1 (Simon et al.,Bio/Technologie 1: 784-791, 1993) mit dem Ligationsansatz transformiert (Hanahan, In. DNA cloning. A practical approach. Vol.1. ILR-Press, Cold Spring Harbor, New York, 1989). Die Selektion auf Plasmid-tragende Zellen erfolgte durch Ausplattieren des Tansformationsansatzes auf LB-Agar (Sambrook et al., Molecular Cloning: a laboratory manual. 2nd Ed. Cold Spring Harbor, New York, 1989), der mit 25 mg/l Kanamycin supplementiert worden war.

Plasmid-DNA wurde aus einer Transformante mit Hilfe des QIAprep Spin Miniprep Kit der Firma Qiagen isoliert und durch Restriktionsspaltung mit den Enzymen SalI und EcoRI und anschließender Agarosegel-Elektrophorese überprüft. Das Plasmid wurde pK18mobsacB_gltAD5V genannt und ist in Figur 1 dargestellt.

### Beispiel 3

### Einbau der Mutation gltAD5V in den Stamm Corynebacterium glutamicum DM1797

Die Mutation gltAD5V soll in den Stamm Corynebacterium glutamicum DM1797 eingebracht werden. Der Stamm DM1797 ist eine Aminoethylcystein-resistente Mutante von Corynebacterium glutamicum ATCC13032 und in der PCT/EP/2005/012417 beschrieben. Sie ist unter der Bezeichnung DSM16833 bei der Deutschen Sammlung für Mikroorganismen und Zellkulturen (DSMZ, Braunschweig, Deutschland) hinterlegt.

Der in Beispiel 2 beschriebene Vektor pK18mobsacB_gltAD5V wurde nach dem Protokoll von Schäfer et al. (Journal of Microbiology 172: 1663-1666 (1990)) in den C. glutamicum Stamm DM1797 durch Konjugation transferiert. Der Vektor kann in DM1797 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_gltAD5V erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18mobsacB_gltAD5V enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, welche die Synthese des für C. glutamicum toxischen Produktes Levan katalysiert. Auf mit Sucrose supplementiertem LB-Agar wachsen daher nur solche Klone, bei denen das integrierte pK18mobsacB_gltAD5V als Folge eines zweiten Rekombinationsereignisses exzisiert wurde. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde ein Klon gesucht, bei dem der gewünschte Austausch, d. h. der Einbau der Mutation gltAD5V, erfolgt war. Hierzu wurde von 10 Klonen mit dem Phänotyp "Wachstum in Gegenwart von Sucrose" und "Nicht-Wachstum in Gegenwart von Kanamycin" die Sequenz des gltA-Gens bestimmt. Auf diese Weise wurde ein Klon identifiziert, der die Mutation gltAD5V trägt. Dieser Stamm wurde als C. glutamicum DM1797_gltAD5V bezeichnet.

### Beispiel 4

### Produktion von L-Lysin

Der in Beispiel 3 erhaltene Stamm DM1797_gltAD5V und der Ausgangsstamm DM1797 wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkulturen wurde das Medium MM verwendet. Die Vorkulturen wurden 24 Stunden bei 33°C und 240 rpm auf einem Schüttler inkubiert. Von diesen Vorkulturen wurde je eine Hauptkultur angeimpft, so dass die Anfangs-OD (660 nm) der Hauptkultur 0,1 OD betrug. Für die Hauptkulturen wurde ebenfalls das Medium MM verwendet.

### Medium MM

| | | |
|---|---|---|
| CSL | 5 | g/l |
| MOPS | 20 | g/l |
| Glucose (getrennt autoklaviert) | 50 | g/l |

### Salze:

| | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 48 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

**Tabelle 1**

| Stamm | OD (660) | Lysin-HCl (g/l) |
|---|---|---|
| DM1797 | 11,9 | 4,8 |
| DM1797_gltAD5V | 11,2 | 5,3 |

### Beispiel 5

### Einbau der Mutation gltAD5V in den Stamm Brevibacterium lactofermentum FERM BP-1763

Die Mutation gltAD5V soll in den Stamm Brevibacterium lactofermentum FERM BP-1763 eingebracht werden. Der Stamm FERM BP-1763 ist ein Mycophenolsäure-resistenter Valin-Produzent (US-A-5,188,948). Er ist auxotroph für L-Isoleucin und L-Methionin.

Der in Beispiel 2 beschriebene Vektor pK18mobsacB_gltAD5V wurde nach dem Protokoll von Schäfer et al. (Journal of Microbiology 172: 1663-1666 (1990)) in den Stamm FERM-BP-1763 durch Konjugation transferiert. Der Vektor kann in FERM BP-1763 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB gltAD5V erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18mobsacB_gltAD5V enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, die die Synthese des für C. glutamicum toxischen Produktes Levan katalysiert. Auf mit Sucrose supplementiertem LB-Agar wachsen daher nur solche Klone, bei denen das integrierte pK18mobsacB_gltAD5V als Folge eines zweiten Rekombinationsereignisses exzisiert wurde. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde ein Klon gesucht, bei dem der gewünschte Austausch, d. h. der Einbau der Mutation gltAD5V, erfolgt war. Hierzu wurde von 10 Klonen mit dem Phänotyp "Wachstum in Gegenwart von Sucrose" und "Nicht-Wachstum in Gegenwart von Kanamycin" die Sequenz des gltA-Gens bestimmt. Auf diese Weise wurde ein Klon identifiziert, der die Mutation gltAD5V trägt. Dieser Stamm wurde als C. glutamicum FERM BP-1763_gltAD5V bezeichnet.

### Beispiel 6

### Produktion von L-Valin

Der in Beispiel 5 erhaltene Stamm FERM BP-1763_gltAD5V und der Ausgangsstamm FERM BP-1763 wurden in einem zur Produktion von Valin geeigneten Nährmedium kultiviert und der Valingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben).

Das Medium CgIII (2.5 g/l NaCl, 10 g/l Bacto-Pepton, 10 g/l Bacto-Yeast Extrakt, pH 7.4, 20 g/l Glukose (getrennt autoklaviert) wurde für die Vorkulturen verwendet. Die Vorkulturen wurden 24 Stunden bei 33°C und 240 rpm auf einem Schüttler inkubiert. Von diesen· Vorkulturen wurde je eine Hauptkultur angeimpft, so dass die Anfangs-OD (660 nm) der Hauptkultur 0,1 OD betrug. Für die Hauptkulturen wurde ebenfalls Medium MM verwendet.

### Medium MM

| | |
|---|---|
| CSL | 5 g/l |
| MOPS | 20 g/l |
| Glucose (getrennt autoklaviert) | 50 g/l |
| Salze: | |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Isoleucin (sterilfiltriert) | 0,1 g/l |
| Methionin (sterilfiltriert) | 0,1 g/l |
| Leucin (sterilfiltriert) | 0,1 g/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 48 Stunden wurde die optische Dichte (OD) bei einer Meßwellenlänge von 660 nm mit dem Biomek 1000 (Beckmann Instruments GmbH, München) bestimmt. Die gebildete Valinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

**Tabelle 2**

| Stamm | OD(660) | Valin (g/l) |
|---|---|---|
| FERM BP-1763 | 8,4 | 11,9 |
| FERM BP-1763_dltAD5V | 7,8 | 12,6 |

### Kurze Beschreibung der Figur:

### Figur 1: Karte des Plasmids pK18mobsacB_gltAD5V

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.
- Kan:: Kanamycin Resistenz-Gen
- SalI:: Schnittstelle des Restriktionsenzyms SalI
- EcoRI:: Schnittstelle des Restriktionsenzyms EcoRI
- gltA':: kloniertes DNA-Fragment enthaltend die Mutation gltAD5V
- sacB:: sacB-Gen
- RP4-mob:: mob-Region mit dem Replikationsursprung für den Transfer (oriT)
- oriV:: Replikationsursprung V

## Patentansprüche

1. Isoliertes Polynukleotid, das für ein Polypeptid kodiert, welches die Aminosäuresequenz von SEQ ID NO:2 umfasst, wobei die L-Asparaginsäure an Position 5 der Aminosäuresequenz durch L-Valin ersetzt ist und wobei das Polypeptid Citratsynthase Aktivität besitzt.

2. Das isolierte Polynukleotid gemäß Anspruch 1, **dadurch gekennzeichnet, dass** das Polypeptid einen bis höchstens fünf konservative Aminosäureaustausch(e) enthält, wobei die Citratsynthase-Aktivität des Polypeptids unverrändert bleibt im Vergleich zur Citratsynthase-Aktivität des Polypeptids gemäß SEQ ID NO: 6.

3. Das isolierte Polynukleotid gemäß Anspruch 1-2, **dadurch gekennzeichnet, dass** das Polynukleotid die Nukleotidsequenz von SEQ ID NO:5 besitzt.

4. Isoliertes Polynukleotid, das eine Nukleotidsequenz umfasst, welche von Position 1 bis 39 die Nukleotidsequenz entsprechend Position 1 bis 39 von SEQ ID NO:11 enthält und von Position 40 bis 105 eine Nukleotidsequenz enthält, die für die Aminosäuresequenz gemäß SEQ ID NO:12 kodiert, wobei an Position 5 L-Valin enthalten ist und das Polynukleotid verwendebar ist für den Einbau der Mutation im gltA-Gen gemäß SEQ ID NO:1 in das Chromosom mittels Allelaustausch.

5. Das isolierte Polynukleotid gemäß Anspruch 4, **dadurch gekennzeichnet, dass** es die Nukleotidsequenz von Position 263 bis 1263 von SEQ ID NO:7 umfasst.

6. Vektor, der ein Polynukleotid gemäß den Ansprüchen 1 bis 5 enthält.

7. Bakterium, das mit dem Vektor gemäß Anspruch 6 transformiert worden ist.

8. Das Bakterium gemäß Anspruch 7, **dadurch gekennzeichnet, dass** es sich um ein Bakterium der Gattung Corynebacterium oder Escherichia,bevorzugt
um Corynebacterium glutamicum oder Escherichia coli handelt.

9. Bakterium der Gattung Corynebacterium, bevorzugt Corynebacterium glutamicum, das ein Polynukleotid gemäß den Ansprüchen 1 bis 5 enthält.

10. Rekombinantes Bakterium der Gattung Corynebacterium welches ein Polynukleotid enthält, das für ein Polypeptid mit Citratsynthase-Aktivität kodiert, welches die Aminosäuresequenz von SEQ ID NO:2 umfasst, wobei an Position 5 der Aminosäuresequenz L-Valin enthalten ist.

11. Das rekombinante Bakterium der Gattung Corynebacterium gemäß Anspruch 10, **dadurch gekennzeichnet, dass** das Polypeptid einen bis höchstens fünf konservative Aminosäuraustausch(e) enthält, wobei die Citratsynthase-Aktivität des Polypeptids unverändert bleibt im Vergleich zur Citratsynthase-Aktivität des Polypeptids gemäß SEQ ID NO: 6.

12. Das rekombinante Bakterium der Gattung Corynebacterium gemäß Ansprüche 10 bis 11, **dadurch gekennzeichnet, dass** das Polynukleotid die Nukleotidsequenz von SEQ ID NO:5 besitzt.

13. Das rekombinante Bakterium der Gattung Corynebacterium gemäß einem oder mehreren der Ansprüche 8 bis 12, **dadurch gekennzeichnet, dass** es ein Polynukleotid enthält, das für ein Polypeptid mit Aspartatkinase-Aktivität kodiert, welches die Aminosäuresequenz von SEQ ID NO:20 umfasst, und einen oder mehrere der Aminosäureaustausche enthält, ausgewählt aus der Gruppe
a) Austausch von L-Alanin an Position 279 des kodierten Aspartatkinaseproteins gemäß SEQ ID NO: 20 gegen L-Threonin (LysC A279T),
b) Austausch von L-Alanin an Position 279 des kodierten Aspartatkinaseproteins gemäß SEQ ID NO: 20 gegen L-Valin (LysC A279V),
c) Austausch von L-Leucin an Position 297 des kodierten Aspartatkinaseproteins gemäß SEQ ID NO: 20 gegen L-Glutamin (LysC L297Q),
d) Austausch von L-Serin an Position 301 des kodierten Aspartatkinaseproteins gemäß SEQ ID NO: 20 gegen L-Phenylalanin (LysC S301F),
e) Austausch von L-Serin an Position 301 des kodierten Aspartatkinaseproteins gemäß SEQ ID NO: 20 gegen L-Tyrosin (LysC S301Y),
f) Austausch von L-Threonin an Position 308 des kodierten Aspartatkinaseproteins gemäß SEQ ID NO: 20 gegen L-Isoleucin (LysC T308I),
g) Austausch von L-Threonin an Position 311 des kodierten Aspartatkinaseproteins gemäß SEQ ID NO: 20 gegen L-Isoleucin (LysC T311I),
h) Austausch von L-Serin an Position 317 des kodierten Aspartatkinaseproteins gemäß SEQ ID NO: 20 gegen L-Alanin (LysC S317A),
i) Austausch von L-Arginin an Position 320 des kodierten Aspartatkinaseproteins gemäß SEQ ID NO: 20 gegen Glycin (LysC R320G),
j) Austausch von Glycin an Position 345 des kodierten Aspartatkinaseproteins gemäß SEQ ID NO: 20 gegen L-Asparaginsäure (LysC G345D),
k) Austausch von L-Threonin an Position 380 des kodierten Aspartatkinaseproteins gemäß SEQ ID NO: 20 gegen L-Isoleucin (LysC T380I), und
l) Austausch von L-Serin an Position 381 des kodierten Aspartatkinaseproteins gemäß SEQ ID NO: 20 gegen L-Phenylalanin (LysC S381F).

14. Das rekombinante Bakterium der Gattung Corynebacterium gemäß Anspruch 13, **dadurch gekennzeichnet, dass** das Polynukleotid, das für ein Polypeptid mit Aspartatkinase-Aktivität kodiert, überexprimiert wird.

15. Das rekombinante Bakterium der Gattung Corynebacterium gemäß einem oder mehreren der Ansprüche 8 bis 14, **dadurch gekennzeichnet, dass** es zusätzlich eines oder mehrere der Merkmale enthält, ausgewählt aus der Gruppe
a) überexprimiertes Polynukleotid, das für eine Dihydrodipicolinat-Synthase (DapA)kodiert,
b) überexprimiertes Polynukleotid, das für eine Aspartatsemialdehyd-Dehydrogenase (Asd) kodiert,
c) überexprimiertes Polynukleotid, das für eine Diaminopimelat-Decarboxylase (LysA) kodiert,
d) überexprimiertes Polynukleotid, das für eine Aspartat-Aminotransferase (Aat) kodiert,
e) überexprimiertes Polynukleotid, das für ein Polypeptid mit L-Lysin-Export Aktivität (LysE) kodiert,
f) ausgeschaltete oder abgeschwächte Aktivität der Malat-Dehydrogenase (Mdh),
g) ausgeschaltete oder abgeschwächte Aktivität der Malat-Chinon Oxidoreduktase (Mqo),
h) überexprimiertes Polynukleotid, das für eine Pyruvat-Carboxylase (Pyc) kodiert, und
i) ausgeschaltete oder abgeschwächte Aktivität der E1p Untereinheit des Pyruvat-Dehydrogenase Komplexes (AceE), vorzugsweise
sämtliche der Merkmale a) bis g) enthält.

16. Das rekombinante Bakterium der Gattung Corynebacterium gemäß einem oder mehreren der Ansprüche 10 bis 15, **dadurch gekennzeichnet, dass** es sich um Corynebacterium glutamicum handelt.

17. Verfahren zur Herstellung einer L-Aminosäure, bevorzugt L-Lysin, L-Valin oder L-Isoleucin, besonders bevorzugt L-Lysin, **dadurch gekennzeichnet, dass** man folgende Schritte durchführt:
a) Fermentation der Bakterien der Gattung Corynebacterium gemäß einem oder mehreren der Ansprüchen 8 bis 16 in einem geeignetem Nährmedium,
b) Akkumulation der L- Aminosäure in dem Nährmedium oder in den Zellen der genannten Bakterien.

18. Verfahren nach Anspruch 17, **dadurch gekennzeichnet, dass** man die L-Aminosäure sammelt und/oder,
dass man die L-Aminosäure isoliert und reinigt.

19. Verfahren nach einem der Ansprüche 17 oder 18, **dadurch gekennzeichnet, dass** Bestandteile der Fermentationsbrühe und/oder Biomasse in ihrer Gesamtheit oder in Anteilen (> 0 bis 100%) im Endprodukt verbleiben.

20. Verfahren nach einem oder mehreren der Ansprüche 17 bis 19, **dadurch gekennzeichnet, dass** es sich um ein Satzverfahren, ein Zulaufverfahren oder um ein kontinuierliches Verfahren handelt.

21. Verfahren nach Anspruch 17, **dadurch gekennzeichnet, dass** man, wenn es sich bei der L-Aminosäure um L-Lysin handelt, einer L-Lysin haltigen Fermentationsbrühe Wasser entzieht und ein Produkt mit einem Wassergehalt von maximal 5 Gew.-% erhält, oder
dass man eine L-Lysin haltige Fermentationsbrühe zunächst konzentriert und anschließend sprühtrocknet oder sprühgranuliert.

22. Verfahren nach Anspruch 17, **dadurch gekennzeichnet, dass**, wenn es sich bei der L-Aminosäure um L-Lysin handelt, man folgende Schritte durchführt
a) den pH-Wert durch Zugabe von Schwefelsäure auf 4,0 bis 5,2 absenkt, und ein molares Sulfat/L-Lysin-Verhältnis von 0,85 bis 1,2 in der Brühe einstellt, gegebenenfalls durch Zugabe von weiteren Sulfat-haltigen Verbindung(n), insbesondere von Ammoniumsulfat und/ oder Ammoniumhydrogensulfat oder Schwefelsäure und Ammoniak in entsprechenden Verhältnissen und
b) das so erhaltene Gemisch durch Wasserentzug aufkonzentriert, und gegebenenfalls granuliert.

## Claims

1. Isolated polynucleotide which codes for a polypeptide that comprises the amino acid sequence of SEQ ID NO: 2, wherein the L-aspartic acid at position 5 of the amino acid sequence is replaced by L-valine and wherein the polypeptide possesses citrate synthase activity.

2. The isolated polynucleotide according to Claim 1, **characterized in that** the polypeptide contains one to at most five conservative amino acid substitution(s), with the citrate synthase activity of the polypeptide remaining unchanged compared to the citrate synthase activity of the polypeptide according to SEQ ID NO: 6.

3. The isolated polynucleotide according to Claim 1-2, **characterized in that** the polynucleotide possesses the nucleotide sequence of SEQ ID NO: 5.

4. Isolated polynucleotide that comprises a nucleotide sequence which contains, from position 1 to 39, the nucleotide sequence corresponding to position 1 to 39 of SEQ ID NO: 11 and contains, from position 40 to 105, a nucleotide sequence that codes for the amino acid sequence according to SEQ ID NO: 12, wherein L-valine is present at position 5 and the polynucleotide can be used for inserting the mutation in the gltA gene according to SEQ ID NO: 1 into the chromosome by means of allelic substitution.

5. The isolated polynucleotide according to Claim 4, **characterized in that** it comprises the nucleotide sequence from position 263 to 1263 of SEQ ID NO: 7.

6. Vector that contains a polynucleotide according to Claims 1 to 5.

7. Bacterium that has been transformed with the vector according to Claim 6.

8. Bacterium according to Claim 7, **characterized in that** it is a bacterium of the genus Corynebacterium or Escherichia, preferably Corynebacterium glutamicum or Escherichia coli.

9. Bacterium of the genus Corynebacterium, preferably Corynebacterium glutamicum, which contains a polynucleotide according to Claims 1 to 5.

10. Recombinant bacterium of the genus Corynebacterium which contains a polynucleotide that codes for a polypeptide with citrate synthase activity, which comprises the amino acid sequence of SEQ ID NO: 2, with L-valine being present at position 5 of the amino acid sequence.

11. The recombinant bacterium of the genus Corynebacterium according to Claim 10, **characterized in that** the polypeptide contains one to at most five conservative amino acid substitution(s), with the citrate synthase activity of the polypeptide remaining unchanged compared to the citrate synthase activity of the polypeptide according to SEQ ID NO: 6.

12. The recombinant bacterium of the genus Corynebacterium according to Claims 10 to 11, **characterized in that** the polynucleotide possesses the nucleotide sequence of SEQ ID NO: 5.

13. The recombinant bacterium of the genus Corynebacterium according to one or more of Claims 8 to 12, **characterized in that** it contains a polynucleotide that codes for a polypeptide with aspartate kinase activity, which comprises the amino acid sequence of SEQ ID NO: 20, and contains one or more of the amino acid substitutions selected from the group
a) substitution of L-alanine at position 279 of the encoded aspartate kinase protein according to SEQ ID NO: 20 for L-threonine (LysC A279T),
b) substitution of L-alanine at position 279 of the encoded aspartate kinase protein according to SEQ ID NO: 20 for L-valine (LysC A279V),
c) substitution of L-leucine at position 297 of the encoded aspartate kinase protein according to SEQ ID NO: 20 for L-glutamine (LysC L297Q),
d) substitution of L-serine at position 301 of the encoded aspartate kinase protein according to SEQ ID NO: 20 for L-phenylalanine (LysC S301F),
e) substitution of L-serine at position 301 of the encoded aspartate kinase protein according to SEQ ID NO: 20 for L-tyrosine (LysC S301Y),
f) substitution of L-threonine at position 308 of the encoded aspartate kinase protein according to SEQ ID NO: 20 for L-isoleucine (LysC T308I),
g) substitution of L-threonine at position 311 of the encoded aspartate kinase protein according to SEQ ID NO: 20 for L-isoleucine (LysC T311I),
h) substitution of L-serine at position 317 of the encoded aspartate kinase protein according to SEQ ID NO: 20 for L-alanine (LysC S317A),
i) substitution of L-arginine at position 320 of the encoded aspartate kinase protein according to SEQ ID NO: 20 for glycine (LysC R320G),
j) substitution of glycine at position 345 of the encoded aspartate kinase protein according to SEQ ID NO: 20 for L-aspartic acid (LysC G345D),
k) substitution of L-threonine at position 380 of the encoded aspartate kinase protein according to SEQ ID NO: 20 for L-isoleucine (LysC T380I), and
l) substitution of L-serine at position 381 of the encoded aspartate kinase protein according to SEQ ID NO: 20 for L-phenylalanine (LysC S381F).

14. The recombinant bacterium of the genus Corynebacterium according to Claim 13, **characterized in that** the polynucleotide that codes for a polypeptide with aspartate kinase activity is overexpressed.

15. The recombinant bacterium of the genus Corynebacterium according to one or more of Claims 8 to 14, **characterized in that** it additionally contains one or more of the characters selected from the group:
a) overexpressed polynucleotide which codes for a dihydrodipicolinate synthase (DapA),
b) overexpressed polynucleotide which codes for an aspartate semialdehyde dehydrogenase (Asd),
c) overexpressed polynucleotide which codes for a diaminopimelate decarboxylase (LysA),
d) overexpressed polynucleotide which codes for an aspartate aminotransferase (Aat),
e) overexpressed polynucleotide which codes for a polypeptide with L-lysine exporting activity (LysE),
f) switched-off or attenuated activity of malate dehydrogenase (Mdh),
g) switched-off or attenuated activity of malate-quinone oxidoreductase (Mqo),
h) overexpressed polynucleotide which codes for a pyruvate carboxylase (Pyc), and
i) switched-off or attenuated activity of the E1p subunit of the pyruvate dehydrogenase complex (AceE), preferably contains all of the characters a) to g) .

16. The recombinant bacterium of the genus Corynebacterium according to one or more of Claims 10 to 15, **characterized in that** it is Corynebacterium glutamicum.

17. Method of production of an L-amino acid, preferably L-lysine, L-valine or L-isoleucine, particularly preferably L-lysine, **characterized in that** the following steps are carried out:
a) fermentation of the bacteria of the genus Corynebacterium according to one or more of Claims 8 to 16 in a suitable nutrient medium,
b) accumulation of the L-amino acid in the nutrient medium or in the cells of said bacteria.

18. Method according to Claim 17, **characterized in that** the L-amino acid is collected and/or **in that** the L-amino acid is isolated and purified.

19. Method according to one of Claims 17 or 18, **characterized in that** constituents of the fermentation broth and/or biomass remain in the end product in their entirety or in proportions (> 0 to 100%).

20. Method according to one or more of Claims 17 to 19, **characterized in that** it is a batch process, a fed batch process or a continuous process.

21. Method according to Claim 17, **characterized in that**, if the L-amino acid is L-lysine, water is extracted from an L-lysine-containing fermentation broth and a product with a maximum water content of 5 wt.% is obtained, or **in that** an L-lysine-containing fermentation broth is first concentrated and then spray-dried or spray-granulated.

22. Method according to Claim 17, **characterized in that**, if the L-amino acid is L-lysine, the following steps are carried out
a) the pH value is lowered to 4.0 to 5.2 by adding sulfuric acid, and a sulfate/L-lysine molar ratio of 0.85 to 1.2 is established in the broth, if necessary by adding further sulfate-containing compound(s), in particular ammonium sulfate and/or ammonium hydrogensulfate or sulfuric acid and ammonia in appropriate proportions and
b) the mixture thus obtained is concentrated by dewatering, and is optionally granulated.

## Revendications

1. Polynucléotide isolé qui code pour un polypeptide qui comprend la séquence d'acides aminés SEQ ID N° 2, l'acide L-asparagique sur la position 5 de la séquence d'acides aminés étant remplacé par la L-valine, et le polypeptide ayant une activité de citrate synthase.

2. Polynucléotide isolé selon la revendication 1, **caractérisé en ce que** le polypeptide contient un à au plus cinq échanges d'acides aminés conservatifs, l'activité de citrate synthase du polypeptide restant inchangée par comparaison avec l'activité de citrate synthase du polypeptide selon SEQ ID N° 6.

3. Polynucléotide isolé selon la revendication 1-2, **caractérisé en ce que** le polynucléotide possède la séquence nucléotidique SEQ ID N° 5.

4. Polynucléotide isolé, qui comprend une séquence nucléotidique qui de la position 1 à la position 39 contient la séquence nucléotidique correspondant aux positions 1 à 39 de SEQ ID N° 11 et, de la position 40 à la position 105, contient une séquence nucléotidique qui code pour la séquence d'acides aminés selon SEQ ID N° 12, une L-valine étant présente sur la position 5, et le polynucléotide pouvant être utilisé pour introduire la mutation dans le gène gltA selon SEQ ID N° 1 dans le chromosome par un échange d'allèles.

5. Polynucléotide isolé selon la revendication 4, **caractérisé en ce qu'**il comprend la séquence nucléotidique de la position 263 à la position 1263 de SEQ ID N° 7.

6. Vecteur, qui contient un polynucléotide selon les revendications 1 à 5.

7. Bactérie, qui a été transformée avec le vecteur selon la revendication 6.

8. Bactérie selon la revendication 7, **caractérisée en ce qu'**il s'agit d'une bactérie du genre Corynebacterium ou Escherichia, de préférence de Corynebacterium glutamicum ou d'Escherichia coli.

9. Bactérie du genre Corynebacterium, de préférence Corynebacterium glutamicum, qui contient un polynucléotide selon les revendications 1 à 5.

10. Bactérie recombinante du genre Corynebacterium, qui contient un polynucléotide qui code pour un polypeptide ayant une activité de citrate synthase, qui comprend la séquence d'acides aminés SEQ ID N° 2 dans laquelle il y a une L-valine sur la position 5 de la séquence d'acides aminés.

11. Bactérie recombinante du genre Corynebacterium selon la revendication 10, **caractérisée en ce que** le polypeptide contient un à au plus cinq échanges d'acides aminés conservatifs, l'activité de citrate synthase du polypeptide restant inchangée par comparaison avec l'activité de citrate synthase du polypeptide selon SEQ ID N° 6.

12. Bactérie recombinante du genre Corynebacterium selon les revendications 10 à 11, **caractérisée en ce que** le polynucléotide possède la séquence nucléotidique SEQ ID N° 5.

13. Bactérie recombinante du genre Corynebacterium selon l'une ou plusieurs des revendications 8 à 12, **caractérisée en ce qu'**elle contient un polynucléotide qui code pour un polypeptide ayant une activité d'aspartate kinase, qui comprend la séquence d'acides aminés SEQ ID N° 20, et qui contient un ou plusieurs échanges d'acides aminés choisis dans le groupe suivant :
a) remplacement de la L-alanine sur la position 279 de la protéine aspartate kinase codée selon SEQ ID N° 20 par la L-thréonine (LysC A279T),
b) remplacement de la L-alanine sur la position 279 de la protéine aspartate kinase codée selon SEQ ID N° 20 par la L-valine (LysC A279V),
c) remplacement de la L-leucine sur la position 297 de la protéine aspartate kinase codée selon SEQ ID N° 20 par la L-glutamine (LysC L297Q),
d) remplacement de la L-sérine sur la position 301 de la protéine aspartate kinase codée selon SEQ ID N° 20 par la L-phénylalanine (LysC S301F),
e) remplacement de la L-sérine sur la position 301 de la protéine aspartate kinase codée selon SEQ ID N° 20 par la L-tyrosine (LysC S301Y),
f) remplacement de la L-thréonine sur la position 308 de la protéine aspartate kinase codée selon SEQ ID N° 20 par la L-isoleucine (LysC T308I),
g) remplacement de la L-thréonine sur la position 311 de la protéine aspartate kinase codée selon SEQ ID N° 20 par la L-isoleucine (LysC T311I),
h) remplacement de la L-sérine sur la position 317 de la protéine aspartate kinase codée selon SEQ ID N° 20 par la L-alanine (LysC S317A),
i) remplacement de la L-arginine sur la position 320 de la protéine aspartate kinase codée selon SEQ ID N° 20 par la glycine (LysC R320G),
j) remplacement de la glycine sur la position 345 de la protéine aspartate kinase codée selon SEQ ID N° 20 par l'acide L-asparagique (LysC G345D),
k) remplacement de la L-thréonine sur la position 380 de la protéine aspartate kinase codée selon SEQ ID N° 20 par la L-isoleucine (LysC T380I), et
l) remplacement de la L-sérine sur la position 381 de la protéine aspartate kinase codée selon SEQ ID N° 20 par la L-phénylalanine (LysC S381F).

14. Bactérie recombinante du genre Corynebacterium selon la revendication 13, **caractérisée en ce que** le polynucléotide qui code pour un polypeptide ayant une activité d'aspartate kinase est surexprimé.

15. Bactérie recombinante du genre Corynebacterium selon l'une ou plusieurs des revendications 8 à 14, **caractérisée en ce qu'**elle contient en outre une ou plusieurs des caractéristiques choisies dans le groupe suivant :
a) un polynucléotide surexprimé, qui code pour une dihydrodipicolinate synthase (DapA),
b) un polynucléotide surexprimé, qui code pour une aspartate semi-aldéhyde déshydrogénase (Asd),
c) un polynucléotide surexprimé, qui code pour une diaminopimélate décarboxylase (LysA),
d) un polynucléotide surexprimé, qui code pour une aspartate-aminotransférase (Aat),
e) un polynucléotide surexprimé, qui code pour un polypeptide ayant une activité d'exportation de la L-lysine (LysE),
f) une activité neutralisée ou affaiblie de malate déshydrogénase (Mdh),
g) une activité neutralisée ou affaiblie de malate-quinone oxydoréductase (Mqo),
h) un polynucléotide surexprimé, qui code pour une pyruvate carboxylase (Pyc), et
i) une activité neutralisée ou affaiblie de la sous-unité E1p du complexe pyruvate-déshydrogénase (AceE),
et de préférence contient la totalité des caractéristiques a) à g) .

16. Bactérie recombinante du genre Corynebacterium selon l'une ou plusieurs des revendications 10 à 15, **caractérisée en ce qu'**il s'agit de Corynebacterium glutamicum.

17. Procédé de préparation d'un acide L-aminé, de préférence la L-lysine, la L-valine ou la L-isoleucine, d'une manière particulièrement préférée la L-lysine, **caractérisé en ce qu'**on procède aux étapes suivantes :
a) fermentation des bactéries du genre Corynebacterium selon l'une ou plusieurs des revendications 8 à 16 dans un milieu nutritif approprié,
b) accumulation de l'acide L-aminé dans le milieu nutritif ou dans les cellules desdites bactéries.

18. Procédé selon la revendication 17, **caractérisé en ce qu'**on collecte l'acide L-aminé, et/ou que l'on isole et purifie l'acide L-aminé.

19. Procédé selon l'une des revendications 17 ou 18, **caractérisé en ce que** les constituants du bouillon de fermentation et/ou de la biomasse restent dans le produit final, en leur totalité ou en partie (> 0 à 100 %).

20. Procédé selon l'une ou plusieurs des revendications 17 à 19, **caractérisé en ce qu'**il s'agit d'un procédé discontinu, d'un procédé discontinu à alimentation programmée ou d'un procédé continu.

21. Procédé selon la revendication 17, **caractérisé en ce que**, quand, pour ce qui concerne l'acide L-aminé, il s'agit de L-lysine, on extrait l'eau d'un bouillon de fermentation contenant la L-lysine, et on obtient un produit ayant une teneur en eau au maximum de 5 % en poids, ou **en ce qu'**on concentre d'abord un bouillon de fermentation contenant la L-lysine, puis on le soumet à un séchage par atomisation ou à une granulation par atomisation.

22. Procédé selon la revendication 17, **caractérisé en ce que**, quand, pour ce qui concerne l'acide L-aminé, il s'agit de L-lysine, on procède aux étapes suivantes :
a) on abaisse à une valeur de 4,0 à 5,2 le pH par addition d'acide sulfurique, et on ajuste dans le bouillon un rapport en moles sulfate/L-lysine de 0,85 à 1,2, éventuellement par addition d'une quantité supplémentaire d'un ou plusieurs composés contenant un sulfate, en particulier de sulfate d'ammonium et/ou d'hydrogénosulfate d'ammonium, ou d'acide sulfurique ou d'ammoniac selon des proportions appropriées, et
b) on concentre par extraction de l'eau le mélange ainsi obtenu, et éventuellement on le granule.


### SEQUENCE LISTING

<110> Degussa GmbH
<120> Verfahren zur Herstellung von L-Aminosäuren
<130> 200600351
<160> 31
<170> PatentIn version 3.3
<210> 1
   <211> 1314
   <212> DNA
   <213> Corynebacterium glutamicum
<220>
   <221> CDS
   <222> (1)..(1311)
   <223> gltA-wildtyp-Gen
<220>
   <221> misc_feature
   <222> (13)..(15)
<400> 1
   atg ttt gaa agg gat atc gtg gct act gat aac aac aag gct gtc ctg      48
   cac tac ccc ggt ggc gag ttc gaa atg gac atc atc gag gct tct gag      96
   ggt aac aac ggt gtt gtc ctg ggc aag atg ctg tct gag act gga ctg     144
   atc act ttt gac cca ggt tat gtg agc act ggc tcc acc gag tcg aag     192
   atc acc tac atc gat ggc gat gcg gga atc ctg cgt tac cgc ggc tat     240
   gac atc gct gat ctg gct gag aat gcc acc ttc aac gag gtt tct tac     288
   cta ctt atc aac ggt gag cta cca acc cca gat gag ctt cac aag ttt     336
   aac gac gag att cgc cac cac acc ctt ctg gac gag gac ttc aag tcc     384
   cag ttc aac gtg ttc cca cgc gac gct cac cca atg gca acc ttg gct     432
   tcc tcg gtt aac att ttg tct acc tac tac cag gac cag ctg aac cca     480
   ctc gat gag gca cag ctt gat aag gca acc gtt cgc ctc atg gca aag     528
   gtt cca atg ctg gct gcg tac gca cac cgc gca cgc aag ggt gct cct       576
   tac atg tac cca gac aac tcc ctc aat gcg cgt gag aac ttc ctg cgc       624
   atg atg ttc ggt tac cca acc gag cca tac gag atc gac cca atc atg       672
   gtc aag gct ctg gac aag ctg ctc atc ctg cac gct gac cac gag cag       720
   aac tgc tcc acc tcc acc gtt cgt atg atc ggt tcc gca cag gcc aac       768
   atg ttt gtc tcc atc gct ggt ggc atc aac gct ctg tcc ggc cca ctg       816
   cac ggt ggc gca aac cag gct gtt ctg gag atg ctc gaa gac atc aag       864
   agc aac cac ggt ggc gac gca acc gag ttc atg aac aag gtc aag aac       912
   aag gaa gac ggc gtc cgc ctc atg ggc ttc gga cac cgc gtt tac aag       960
   aac tac gat cca cgt gca gca atc gtc aag gag acc gca cac gag atc      1008
   ctc gag cac ctc ggt ggc gac gat ctt ctg gat ctg gca atc aag ctg      1056
   gaa gaa att gca ctg gct gat gat tac ttc atc tcc cgc aag ctc tac      1104
   ccg aac gta gac ttc tac acc ggc ctg atc tac cgc gca atg ggc ttc      1152
   cca act gac ttc ttc acc gta ttg ttc gca atc ggt cgt ctg cca gga      1200
   tgg atc gct cac tac cgc gag cag ctc ggt gca gca ggc aac aag atc      1248
   aac cgc cca cgc cag gtc tac acc ggc aac gaa tcc cgc aag ttg gtt      1296
   cct cgc gag gag cgc taa                                              1314
<210> 2
   <211> 437
   <212> PRT
   <213> Corynebacterium glutamicum
<400> 2
<210> 3
   <211> 2814
   <212> DNA
   <213> Corynebacterium glutamicum
<220>
   <221> misc_feature
   <222> (1)..(750)
   <223> stromaufwärts der Kodierregion gelegene Sequenz
<220>
   <221> CDS
   <222> (751)..(2061)
   <223> gltA-wildtyp-Gen
<220>
   <221> misc_feature
   <222> (763)..(765)
<220>
   <221> misc_feature
   <222> (2062)..(2814)
   <223> stromabwärts der kodierregion gelegene Sequenz
<400> 3
   agggcagggt ggggaagtcg gtcatgtctt cgggcaactt tctgcgcttg gaagtaaaag      60
   ggccagggat cgttaacgat ctgacccaac aactataacc ctgaagctgt cagttcctag     120
   caccctagat tcttcacgca gtctcccaaa cgatgaaaaa cgcccaaaac tggcgacacc     180
   gaactattga aaacgcgggg attagttgac cagccaccaa tttgggggta gctcaaagtt     240
   ttgcaaagtt ttcaatttct aggttgttaa tatcccctga ggttgcgtta tagggtggcg     300
   aattgcatgg ggaaagctac tcggcaccca tccttgtcgc gtgcatcaca aactttgcta     360
   aactgtgcac cagtccactt attgtgggat ttttaatgcc ttaaaggcca gcattttcac     420
   cctctagcgg ggttgaatgc tggccttgag ggtgcagaac taaatagcag cacatcggca     480
   caattgatct gagttctatt ggcgtgaccg tggctactga ttacggtggc tgtgggtggt     540
   cgggaatgat gtaaccaacg tgattgtggg ggaattggct ctcacttcgg atatggctaa     600
   accgcattta tcggtatagc gtgttaaccg gaccagattg ggaaagaaat gtgtcgagta     660
   acaaaaactg acatgcgctt ggcgcatccc agttggtaag aataaacggg actacttccg     720
   taatccggaa gagttttttt ccgaacaaat atg ttt gaa agg gat atc gtg gct      774
   act gat aac aac aag gct gtc ctg cac tac ccc ggt ggc gag ttc gaa      822
   atg gac atc atc gag gct tct gag ggt aac aac ggt gtt gtc ctg ggc      870
   aag atg ctg tct gag act gga ctg atc act ttt gac cca ggt tat gtg      918
   agc act ggc tcc acc gag tcg aag atc acc tac atc gat ggc gat gcg      966
   gga atc ctg cgt tac cgc ggc tat gac atc gct gat ctg gct gag aat     1014
   gcc acc ttc aac gag gtt tct tac cta ctt atc aac ggt gag cta cca     1062
   acc cca gat gag ctt cac aag ttt aac gac gag att cgc cac cac acc     1110
   ctt ctg gac gag gac ttc aag tcc cag ttc aac gtg ttc cca cgc gac     1158
   gct cac cca atg gca acc ttg gct tcc tcg gtt aac att ttg tct acc      1206
   tac tac cag gac cag ctg aac cca ctc gat gag gca cag ctt gat aag      1254
   gca acc gtt cgc ctc atg gca aag gtt cca atg ctg gct gcg tac gca      1302
   cac cgc gca cgc aag ggt gct cct tac atg tac cca gac aac tcc ctc      1350
   aat gcg cgt gag aac ttc ctg cgc atg atg ttc ggt tac cca acc gag      1398
   cca tac gag atc gac cca atc atg gtc aag gct ctg gac aag ctg ctc      1446
   atc ctg cac gct gac cac gag cag aac tgc tcc acc tcc acc gtt cgt      1494
   atg atc ggt tcc gca cag gcc aac atg ttt gtc tcc atc gct ggt ggc      1542
   atc aac gct ctg tcc ggc cca ctg cac ggt ggc gca aac cag gct gtt      1590
   ctg gag atg ctc gaa gac atc aag agc aac cac ggt ggc gac gca acc      1638
   gag ttc atg aac aag gtc aag aac aag gaa gac ggc gtc cgc ctc atg      1686
   ggc ttc gga cac cgc gtt tac aag aac tac gat cca cgt gca gca atc      1734
   gtc aag gag acc gca cac gag atc ctc gag cac ctc ggt ggc gac gat      1782
   ctt ctg gat ctg gca atc aag ctg gaa gaa att gca ctg gct gat gat      1830
   tac ttc atc tcc cgc aag ctc tac ccg aac gta gac ttc tac acc ggc      1878
   ctg atc tac cgc gca atg ggc ttc cca act gac ttc ttc acc gta ttg      1926
   ttc gca atc ggt cgt ctg cca gga tgg atc gct cac tac cgc gag cag      1974
   ctc ggt gca gca ggc aac aag atc aac cgc cca cgc cag gtc tac acc      2022
   ggc aac gaa tcc cgc aag ttg gtt cct cgc gag gag cgc taaatttagc       2071
   ggatgattct cgttcaactt cggccgaagc cacttcgtct gtcataatga cagggatggt    2131
   ttcggccgtt tttgcatgaa accaaaaaat acgattttca aggagcatgt acagcacatg    2191
   gaaaagccac agattgagct accggtcggt ccagcaccgg aagatctcgt aatctctgac    2251
   atcatcgttg gcgaaggagc agaagcccgc ccaggtggag aagttgaggt ccactatgtg    2311
   ggcgttgact ttgaaaccgg cgaggagttt gactcttcct gggatcgtgg acagaccagc    2371
   cagttcccac tcaacggcct cattgcaggt tggcaagagg gaattccagg catgaaggtc    2431
   ggcggacgtc gtcagctgac cattccgcca gaggctgctt acggccctga gggttccggc    2491
   cacccactgt ctggccgtac cctggtgttc atcatcgatt tgatcagcgc ataattttct    2551
   ttactgcgct aaacgctcaa atcgtgtgaa gcgactgtcg cgtcccgccc tctccggatt    2611
   gttatccaat tcggagaggg cgttgctgat tgtgccgaga atttcttcaa caaagtgctc    2671
   ggtttcggcg acgatcccgt cgataagccc ttggcttaaa agtgcgtgcg cctgcacgcc    2731
   ttgtcgctct atgatttccg cggcgtggtt ggtgtcgcgg aagaggatgg ccgaggcgcc    2791
   ctctggtggc aatgcggaca gcc                                            2814
<210> 4
   <211> 437
   <212> PRT
   <213> Corynebacterium glutamicum
<400> 4
<210> 5
   <211> 1314
   <212> DNA
   <213> Corynebacterium glutamicum
<220>
   <221> CDS
   <222> (1)..(1311)
   <223> gltA-Allel
<220>
   <221> misc_feature
   <222> (13)..(15)
<220>
   <221> mutation
   <222> (14)..(14)
   <223> Transversion A > T
<400> 5
   atg ttt gaa agg gtt atc gtg gct act gat aac aac aag gct gtc ctg      48
   Met Phe Glu Arg Val Ile Val Ala Thr Asp Asn Asn Lys Ala Val Leu
   1               5                   10                  15
   cac tac ccc ggt ggc gag ttc gaa atg gac atc atc gag gct tct gag      96
   His Tyr Pro Gly Gly Glu Phe Glu Met Asp Ile Ile Glu Ala Ser Glu
               20                  25                  30
   ggt aac aac ggt gtt gtc ctg ggc aag atg ctg tct gag act gga ctg     144
   Gly Asn Asn Gly Val Val Leu Gly Lys Met Leu Ser Glu Thr Gly Leu
           35                  40                  45
   atc act ttt gac cca ggt tat gtg agc act ggc tcc acc gag tcg aag     192
   Ile Thr Phe Asp Pro Gly Tyr Val Ser Thr Gly Ser Thr Glu Ser Lys
       50                  55                  60
   atc acc tac atc gat ggc gat gcg gga atc ctg cgt tac cgc ggc tat     240
   Ile Thr Tyr Ile Asp Gly Asp Ala Gly Ile Leu Arg Tyr Arg Gly Tyr
   65                  70                  75                  80
   gac atc gct gat ctg gct gag aat gcc acc ttc aac gag gtt tct tac     288
   Asp Ile Ala Asp Leu Ala Glu Asn Ala Thr Phe Asn Glu Val Ser Tyr
                   85                  90                  95
   cta ctt atc aac ggt gag cta cca acc cca gat gag ctt cac aag ttt     336
   Leu Leu Ile Asn Gly Glu Leu Pro Thr Pro Asp Glu Leu His Lys Phe
               100                 105                 110
   aac gac gag att cgc cac cac acc ctt ctg gac gag gac ttc aag tcc     384
   Asn Asp Glu Ile Arg His His Thr Leu Leu Asp Glu Asp Phe Lys Ser
           115                 120                 125
   cag ttc aac gtg ttc cca cgc gac gct cac cca atg gca acc ttg gct      432
   Gln Phe Asn Val Phe Pro Arg Asp Ala His Pro Met Ala Thr Leu Ala
       130                 135                 140
   tcc tcg gtt aac att ttg tct acc tac tac cag gac cag ctg aac cca      480
   Ser Ser Val Asn Ile Leu Ser Thr Tyr Tyr Gln Asp Gln Leu Asn Pro
   145                 150                 155                 160
   ctc gat gag gca cag ctt gat aag gca acc gtt cgc ctc atg gca aag      528
   Leu Asp Glu Ala Gln Leu Asp Lys Ala Thr Val Arg Leu Met Ala Lys
                   165                 170                 175
   gtt cca atg ctg gct gcg tac gca cac cgc gca cgc aag ggt gct cct      576
   Val Pro Met Leu Ala Ala Tyr Ala His Arg Ala Arg Lys Gly Ala Pro
               180                 185                 190
   tac atg tac cca gac aac tcc ctc aat gcg cgt gag aac ttc ctg cgc      624
   Tyr Met Tyr Pro Asp Asn Ser Leu Asn Ala Arg Glu Asn Phe Leu Arg
           195                 200                 205
   atg atg ttc ggt tac cca acc gag cca tac gag atc gac cca atc atg      672
   Met Met Phe Gly Tyr Pro Thr Glu Pro Tyr Glu Ile Asp Pro Ile Met
       210                 215                 220
   gtc aag gct ctg gac aag ctg ctc atc ctg cac gct gac cac gag cag      720
   Val Lys Ala Leu Asp Lys Leu Leu Ile Leu His Ala Asp His Glu Gln
   225                 230                 235                 240
   aac tgc tcc acc tcc acc gtt cgt atg atc ggt tcc gca cag gcc aac      768
   Asn Cys Ser Thr Ser Thr Val Arg Met Ile Gly Ser Ala Gln Ala Asn
                   245                 250                 255
   atg ttt gtc tcc atc gct ggt ggc atc aac gct ctg tcc ggc cca ctg      816
   Met Phe Val Ser Ile Ala Gly Gly Ile Asn Ala Leu Ser Gly Pro Leu
               260                 265                 270
   cac ggt ggc gca aac cag gct gtt ctg gag atg ctc gaa gac atc aag      864
   His Gly Gly Ala Asn Gln Ala Val Leu Glu Met Leu Glu Asp Ile Lys
           275                 280                 285
   agc aac cac ggt ggc gac gca acc gag ttc atg aac aag gtc aag aac      912
   Ser Asn His Gly Gly Asp Ala Thr Glu Phe Met Asn Lys Val Lys Asn
       290                 295                 300
   aag gaa gac ggc gtc cgc ctc atg ggc ttc gga cac cgc gtt tac aag      960
   Lys Glu Asp Gly Val Arg Leu Met Gly Phe Gly His Arg Val Tyr Lys
   305                 310                 315                 320
   aac tac gat cca cgt gca gca atc gtc aag gag acc gca cac gag atc     1008
   Asn Tyr Asp Pro Arg Ala Ala Ile Val Lys Glu Thr Ala His Glu Ile
                   325                 330                 335
   ctc gag cac ctc ggt ggc gac gat ctt ctg gat ctg gca atc aag ctg     1056
   Leu Glu His Leu Gly Gly Asp Asp Leu Leu Asp Leu Ala Ile Lys Leu
               340                 345                 350
   gaa gaa att gca ctg gct gat gat tac ttc atc tcc cgc aag ctc tac     1104
   Glu Glu Ile Ala Leu Ala Asp Asp Tyr Phe Ile Ser Arg Lys Leu Tyr
           355                 360                 365
   ccg aac gta gac ttc tac acc ggc ctg atc tac cgc gca atg ggc ttc     1152
   Pro Asn Val Asp Phe Tyr Thr Gly Leu Ile Tyr Arg Ala Met Gly Phe
       370                 375                 380
   cca act gac ttc ttc acc gta ttg ttc gca atc ggt cgt ctg cca gga     1200
   Pro Thr Asp Phe Phe Thr Val Leu Phe Ala Ile Gly Arg Leu Pro Gly
   385                 390                 395                 400
   tgg atc gct cac tac cgc gag cag ctc ggt gca gca ggc aac aag atc     1248
   Trp Ile Ala His Tyr Arg Glu Gln Leu Gly Ala Ala Gly Asn Lys Ile
                   405                 410                 415
   aac cgc cca cgc cag gtc tac acc ggc aac gaa tcc cgc aag ttg gtt        1296
   Asn Arg Pro Arg Gln Val Tyr Thr Gly Asn Glu Ser Arg Lys Leu Val
               420                 425                 430
   cct cgc gag gag cgc taa                                                1314
   Pro Arg Glu Glu Arg
           435
<210> 6
   <211> 437
   <212> PRT
   <213> Corynebacterium glutamicum
<400> 6
   Met Phe Glu Arg Val Ile Val Ala Thr Asp Asn Asn Lys Ala Val Leu
   1               5                   10                  15
   His Tyr Pro Gly Gly Glu Phe Glu Met Asp Ile Ile Glu Ala Ser Glu
               20                  25                  30
   Gly Asn Asn Gly Val Val Leu Gly Lys Met Leu Ser Glu Thr Gly Leu
           35                  40                  45
   Ile Thr Phe Asp Pro Gly Tyr Val Ser Thr Gly Ser Thr Glu Ser Lys
       50                  55                  60
   Ile Thr Tyr Ile Asp Gly Asp Ala Gly Ile Leu Arg Tyr Arg Gly Tyr
   65                  70                  75                  80
   Asp Ile Ala Asp Leu Ala Glu Asn Ala Thr Phe Asn Glu Val Ser Tyr
                   85                  90                  95
   Leu Leu Ile Asn Gly Glu Leu Pro Thr Pro Asp Glu Leu His Lys Phe
               100                 105                 110
   Asn Asp Glu Ile Arg His His Thr Leu Leu Asp Glu Asp Phe Lys Ser
           115                 120                 125
   Gln Phe Asn Val Phe Pro Arg Asp Ala His Pro Met Ala Thr Leu Ala
       130                 135                 140
   Ser Ser Val Asn Ile Leu Ser Thr Tyr Tyr Gln Asp Gln Leu Asn Pro
   145                 150                 155                 160
   Leu Asp Glu Ala Gln Leu Asp Lys Ala Thr Val Arg Leu Met Ala Lys
                   165                 170                 175
   Val Pro Met Leu Ala Ala Tyr Ala His Arg Ala Arg Lys Gly Ala Pro
               180                 185                 190
   Tyr Met Tyr Pro Asp Asn Ser Leu Asn Ala Arg Glu Asn Phe Leu Arg
           195                 200                 205
   Met Met Phe Gly Tyr Pro Thr Glu Pro Tyr Glu Ile Asp Pro Ile Met
       210                 215                 220
   Val Lys Ala Leu Asp Lys Leu Leu Ile Leu His Ala Asp His Glu Gln
   225                 230                 235                 240
   Asn Cys Ser Thr Ser Thr Val Arg Met Ile Gly Ser Ala Gln Ala Asn
                   245                 250                 255
   Met Phe Val Ser Ile Ala Gly Gly Ile Asn Ala Leu Ser Gly Pro Leu
               260                 265                 270
   His Gly Gly Ala Asn Gln Ala Val Leu Glu Met Leu Glu Asp Ile Lys
           275                 280                 285
   Ser Asn His Gly Gly Asp Ala Thr Glu Phe Met Asn Lys Val Lys Asn
       290                 295                 300
   Lys Glu Asp Gly Val Arg Leu Met Gly Phe Gly His Arg Val Tyr Lys
   305                 310                 315                 320
   Asn Tyr Asp Pro Arg Ala Ala Ile Val Lys Glu Thr Ala His Glu Ile
                   325                 330                 335
   Leu Glu His Leu Gly Gly Asp Asp Leu Leu Asp Leu Ala Ile Lys Leu
               340                 345                 350
   Glu Glu Ile Ala Leu Ala Asp Asp Tyr Phe Ile Ser Arg Lys Leu Tyr
           355                 360                 365
   Pro Asn Val Asp Phe Tyr Thr Gly Leu Ile Tyr Arg Ala Met Gly Phe
       370                 375                 380
   Pro Thr Asp Phe Phe Thr Val Leu Phe Ala Ile Gly Arg Leu Pro Gly
   385                 390                 395                 400
   Trp Ile Ala His Tyr Arg Glu Gln Leu Gly Ala Ala Gly Asn Lys Ile
                   405                 410                 415
   Asn Arg Pro Arg Gln Val Tyr Thr Gly Asn Glu Ser Arg Lys Leu Val
               420                 425                 430
   Pro Arg Glu Glu Arg
           435
<210> 7
   <211> 2814
   <212> DNA
   <213> Corynebacterium glutamicum
<220>
   <221> misc_feature
   <222> (1)..(750)
   <223> stromaufwärts der Kodierregion gelegene Sequenz
<220>
   <221> CDS
   <222> (751)..(2061)
   <223> gltA-Allel
<220>
   <221> misc_feature
   <222> (763)..(765)
<220>
   <221> mutation
   <222> (764)..(764)
   <223> A > T Transversion
<220>
   <221> misc_feature
   <222> (2062)..(2814)
   <223> stromabwärts der Kodierregion gelegene Sequenz
<400> 7
   agggcagggt ggggaagtcg gtcatgtctt cgggcaactt tctgcgcttg gaagtaaaag      60
   ggccagggat cgttaacgat ctgacccaac aactataacc ctgaagctgt cagttcctag     120
   caccctagat tcttcacgca gtctcccaaa cgatgaaaaa cgcccaaaac tggcgacacc     180
   gaactattga aaacgcgggg attagttgac cagccaccaa tttgggggta gctcaaagtt     240
   ttgcaaagtt ttcaatttct aggttgttaa tatcccctga ggttgcgtta tagggtggcg     300
   aattgcatgg ggaaagctac tcggcaccca tccttgtcgc gtgcatcaca aactttgcta     360
   aactgtgcac cagtccactt attgtgggat ttttaatgcc ttaaaggcca gcattttcac     420
   cctctagcgg ggttgaatgc tggccttgag ggtgcagaac taaatagcag cacatcggca     480
   caattgatct gagttctatt ggcgtgaccg tggctactga ttacggtggc tgtgggtggt     540
   cgggaatgat gtaaccaacg tgattgtggg ggaattggct ctcacttcgg atatggctaa     600
   accgcattta tcggtatagc gtgttaaccg gaccagattg ggaaagaaat gtgtcgagta     660
   acaaaaactg acatgcgctt ggcgcatccc agttggtaag aataaacggg actacttccg     720
   taatccggaa gagttttttt ccgaacaaat atg ttt gaa agg gtt atc gtg gct      774
                                    Met Phe Glu Arg Val Ile Val Ala
                                    1               5
   act gat aac aac aag gct gtc ctg cac tac ccc ggt ggc gag ttc gaa       822
   Thr Asp Asn Asn Lys Ala Val Leu His Tyr Pro Gly Gly Glu Phe Glu
       10                  15                  20
   atg gac atc atc gag gct tct gag ggt aac aac ggt gtt gtc ctg ggc       870
   Met Asp Ile Ile Glu Ala Ser Glu Gly Asn Asn Gly Val Val Leu Gly
   25                  30                  35                  40
   aag atg ctg tct gag act gga ctg atc act ttt gac cca ggt tat gtg       918
   Lys Met Leu Ser Glu Thr Gly Leu Ile Thr Phe Asp Pro Gly Tyr Val
                   45                  50                  55
   agc act ggc tcc acc gag tcg aag atc acc tac atc gat ggc gat gcg       966
   Ser Thr Gly Ser Thr Glu Ser Lys Ile Thr Tyr Ile Asp Gly Asp Ala
               60                  65                  70
   gga atc ctg cgt tac cgc ggc tat gac atc gct gat ctg gct gag aat      1014
   Gly Ile Leu Arg Tyr Arg Gly Tyr Asp Ile Ala Asp Leu Ala Glu Asn
   75 80 85
   gcc acc ttc aac gag gtt tct tac cta ctt atc aac ggt gag cta cca 1062
   Ala Thr Phe Asn Glu Val Ser Tyr Leu Leu Ile Asn Gly Glu Leu Pro
   90 95 100
   acc cca gat gag ctt cac aag ttt aac gac gag att cgc cac cac acc 1110
   Thr Pro Asp Glu Leu His Lys Phe Asn Asp Glu Ile Arg His His Thr
   105 110 115 120
   ctt ctg gac gag gac ttc aag tcc cag ttc aac gtg ttc cca cgc gac 1158
   Leu Leu Asp Glu Asp Phe Lys Ser Gln Phe Asn Val Phe Pro Arg Asp
   125 130 135
   gct cac cca atg gca acc ttg gct tcc tcg gtt aac att ttg tct acc 1206
   Ala His Pro Met Ala Thr Leu Ala Ser Ser Val Asn Ile Leu Ser Thr
   140 145 150
   tac tac cag gac cag ctg aac cca ctc gat gag gca cag ctt gat aag 1254
   Tyr Tyr Gln Asp Gln Leu Asn Pro Leu Asp Glu Ala Gln Leu Asp Lys
   155 160 165
   gca acc gtt cgc ctc atg gca aag gtt cca atg ctg gct gcg tac gca 1302
   Ala Thr Val Arg Leu Met Ala Lys Val Pro Met Leu Ala Ala Tyr Ala
   170 175 180
   cac cgc gca cgc aag ggt gct cct tac atg tac cca gac aac tcc ctc 1350
   His Arg Ala Arg Lys Gly Ala Pro Tyr Met Tyr Pro Asp Asn Ser Leu
   185 190 195 200
   aat gcg cgt gag aac ttc ctg cgc atg atg ttc ggt tac cca acc gag 1398
   Asn Ala Arg Glu Asn Phe Leu Arg Met Met Phe Gly Tyr Pro Thr Glu
   205 210 215
   cca tac gag atc gac cca atc atg gtc aag gct ctg gac aag ctg ctc 1446
   Pro Tyr Glu Ile Asp Pro Ile Met Val Lys Ala Leu Asp Lys Leu Leu
   220 225 230
   atc ctg cac gct gac cac gag cag aac tgc tcc acc tcc acc gtt cgt 1494
   Ile Leu His Ala Asp His Glu Gln Asn Cys Ser Thr Ser Thr Val Arg
   235 240 245
   atg atc ggt tcc gca cag gcc aac atg ttt gtc tcc atc gct ggt ggc 1542
   Met Ile Gly Ser Ala Gln Ala Asn Met Phe Val Ser Ile Ala Gly Gly
   250 255 260
   atc aac gct ctg tcc ggc cca ctg cac ggt ggc gca aac cag gct gtt 1590
   Ile Asn Ala Leu Ser Gly Pro Leu His Gly Gly Ala Asn Gln Ala Val
   265 270 275 280
   ctg gag atg ctc gaa gac atc aag agc aac cac ggt ggc gac gca acc 1638
   Leu Glu Met Leu Glu Asp Ile Lys Ser Asn His Gly Gly Asp Ala Thr
   285 290 295
   gag ttc atg aac aag gtc aag aac aag gaa gac ggc gtc cgc ctc atg 1686
   Glu Phe Met Asn Lys Val Lys Asn Lys Glu Asp Gly Val Arg Leu Met
   300 305 310
   ggc ttc gga cac cgc gtt tac aag aac tac gat cca cgt gca gca atc 1734
   Gly Phe Gly His Arg Val Tyr Lys Asn Tyr Asp Pro Arg Ala Ala Ile
   315 320 325
   gtc aag gag acc gca cac gag atc ctc gag cac ctc ggt ggc gac gat 1782
   Val Lys Glu Thr Ala His Glu Ile Leu Glu His Leu Gly Gly Asp Asp
   330 335 340
   ctt ctg gat ctg gca atc aag ctg gaa gaa att gca ctg gct gat gat 1830
   Leu Leu Asp Leu Ala Ile Lys Leu Glu Glu Ile Ala Leu Ala Asp Asp
   345                 350                 355                 360
   tac ttc atc tcc cgc aag ctc tac ccg aac gta gac ttc tac acc ggc     1878
   Tyr Phe Ile Ser Arg Lys Leu Tyr Pro Asn Val Asp Phe Tyr Thr Gly
                   365                 370                 375
   ctg atc tac cgc gca atg ggc ttc cca act gac ttc ttc acc gta ttg     1926
   Leu Ile Tyr Arg Ala Met Gly Phe Pro Thr Asp Phe Phe Thr Val Leu
               380                 385                 390
   ttc gca atc ggt cgt ctg cca gga tgg atc gct cac tac cgc gag cag     1974
   Phe Ala Ile Gly Arg Leu Pro Gly Trp Ile Ala His Tyr Arg Glu Gln
           395                 400                 405
   ctc ggt gca gca ggc aac aag atc aac cgc cca cgc cag gtc tac acc     2022
   Leu Gly Ala Ala Gly Asn Lys Ile Asn Arg Pro Arg Gln Val Tyr Thr
       410                 415                 420
   ggc aac gaa tcc cgc aag ttg gtt cct cgc gag gag cgc taaatttagc      2071
   Gly Asn Glu Ser Arg Lys Leu Val Pro Arg Glu Glu Arg
   425                 430                 435
   ggatgattct cgttcaactt cggccgaagc cacttcgtct gtcataatga cagggatggt   2131
   ttcggccgtt tttgcatgaa accaaaaaat acgattttca aggagcatgt acagcacatg   2191
   gaaaagccac agattgagct accggtcggt ccagcaccgg aagatctcgt aatctctgac   2251
   atcatcgttg gcgaaggagc agaagcccgc ccaggtggag aagttgaggt ccactatgtg   2311
   ggcgttgact ttgaaaccgg cgaggagttt gactcttcct gggatcgtgg acagaccagc   2371
   cagttcccac tcaacggcct cattgcaggt tggcaagagg gaattccagg catgaaggtc   2431
   ggcggacgtc gtcagctgac cattccgcca gaggctgctt acggccctga gggttccggc   2491
   cacccactgt ctggccgtac cctggtgttc atcatcgatt tgatcagcgc ataattttct   2551
   ttactgcgct aaacgctcaa atcgtgtgaa gcgactgtcg cgtcccgccc tctccggatt   2611
   gttatccaat tcggagaggg cgttgctgat tgtgccgaga atttcttcaa caaagtgctc   2671
   ggtttcggcg acgatcccgt cgataagccc ttggcttaaa agtgcgtgcg cctgcacgcc   2731
   ttgtcgctct atgatttccg cggcgtggtt ggtgtcgcgg aagaggatgg ccgaggcgcc   2791
   ctctggtggc aatgcggaca gcc                                           2814
<210> 8
   <211> 437
   <212> PRT
   <213> Corynebacterium glutamicum
<400> 8
   Met Phe Glu Arg Val Ile Val Ala Thr Asp Asn Asn Lys Ala Val Leu
   1               5                   10                  15
   His Tyr Pro Gly Gly Glu Phe Glu Met Asp Ile Ile Glu Ala Ser Glu
               20                  25                  30
   Gly Asn Asn Gly Val Val Leu Gly Lys Met Leu Ser Glu Thr Gly Leu
           35                  40                  45
   Ile Thr Phe Asp Pro Gly Tyr Val Ser Thr Gly Ser Thr Glu Ser Lys
   50 55 60
   Ile Thr Tyr Ile Asp Gly Asp Ala Gly Ile Leu Arg Tyr Arg Gly Tyr
   65 70 75 80
   Asp Ile Ala Asp Leu Ala Glu Asn Ala Thr Phe Asn Glu Val Ser Tyr
   85 90 95
   Leu Leu Ile Asn Gly Glu Leu Pro Thr Pro Asp Glu Leu His Lys Phe
   100 105 110
   Asn Asp Glu Ile Arg His His Thr Leu Leu Asp Glu Asp Phe Lys Ser
   115 120 125
   Gln Phe Asn Val Phe Pro Arg Asp Ala His Pro Met Ala Thr Leu Ala
   130 135 140
   Ser Ser Val Asn Ile Leu Ser Thr Tyr Tyr Gln Asp Gln Leu Asn Pro
   145 150 155 160
   Leu Asp Glu Ala Gln Leu Asp Lys Ala Thr Val Arg Leu Met Ala Lys
   165 170 175
   Val Pro Met Leu Ala Ala Tyr Ala His Arg Ala Arg Lys Gly Ala Pro
   180 185 190
   Tyr Met Tyr Pro Asp Asn Ser Leu Asn Ala Arg Glu Asn Phe Leu Arg
   195 200 205
   Met Met Phe Gly Tyr Pro Thr Glu Pro Tyr Glu Ile Asp Pro Ile Met
   210 215 220
   Val Lys Ala Leu Asp Lys Leu Leu Ile Leu His Ala Asp His Glu Gln
   225 230 235 240
   Asn Cys Ser Thr Ser Thr Val Arg Met Ile Gly Ser Ala Gln Ala Asn
   245 250 255
   Met Phe Val Ser Ile Ala Gly Gly Ile Asn Ala Leu Ser Gly Pro Leu
   260 265 270
   His Gly Gly Ala Asn Gln Ala Val Leu Glu Met Leu Glu Asp Ile Lys
   275 280 285
   Ser Asn His Gly Gly Asp Ala Thr Glu Phe Met Asn Lys Val Lys Asn
   290 295 300
   Lys Glu Asp Gly Val Arg Leu Met Gly Phe Gly His Arg Val Tyr Lys
   305 310 315 320
   Asn Tyr Asp Pro Arg Ala Ala Ile Val Lys Glu Thr Ala His Glu Ile
   325 330 335
   Leu Glu His Leu Gly Gly Asp Asp Leu Leu Asp Leu Ala Ile Lys Leu
   340 345 350
   Glu Glu Ile Ala Leu Ala Asp Asp Tyr Phe Ile Ser Arg Lys Leu Tyr
   355 360 365
   Pro Asn Val Asp Phe Tyr Thr Gly Leu Ile Tyr Arg Ala Met Gly Phe
   370 375 380
   Pro Thr Asp Phe Phe Thr Val Leu Phe Ala Ile Gly Arg Leu Pro Gly
   385 390 395 400
   Trp Ile Ala His Tyr Arg Glu Gln Leu Gly Ala Ala Gly Asn Lys Ile
   405 410 415
   Asn Arg Pro Arg Gln Val Tyr Thr Gly Asn Glu Ser Arg Lys Leu Val
   420 425 430
   Pro Arg Glu Glu Arg
   435
<210> 9
   <211> 2814
   <212> DNA
   <213> Corynebacterium glutamicum
<220>
   <221> misc_feature
   <222> (1)..(750)
   <223> stromaufwärts der Kodierregion gelegene Sequenz
<220>
   <221> CDS
   <222> (751)..(2061)
   <223> gltA-Allel
<220>
   <221> misc_feature
   <222> (763)..(765)
   <223> n is a, c, g, or t
<220>
   <221> misc_feature
   <222> (2062)..(2814)
   <223> stromabwärts der Kodierregion gelegene Sequenz
<400> 9
   agggcagggt ggggaagtcg gtcatgtctt cgggcaactt tctgcgcttg gaagtaaaag     60
   ggccagggat cgttaacgat ctgacccaac aactataacc ctgaagctgt cagttcctag    120
   caccctagat tcttcacgca gtctcccaaa cgatgaaaaa cgcccaaaac tggcgacacc    180
   gaactattga aaacgcgggg attagttgac cagccaccaa tttgggggta gctcaaagtt    240
   ttgcaaagtt ttcaatttct aggttgttaa tatcccctga ggttgcgtta tagggtggcg    300
   aattgcatgg ggaaagctac tcggcaccca tccttgtcgc gtgcatcaca aactttgcta    360
   aactgtgcac cagtccactt attgtgggat ttttaatgcc ttaaaggcca gcattttcac      420
   cctctagcgg ggttgaatgc tggccttgag ggtgcagaac taaatagcag cacatcggca      480
   caattgatct gagttctatt ggcgtgaccg tggctactga ttacggtggc tgtgggtggt      540
   cgggaatgat gtaaccaacg tgattgtggg ggaattggct ctcacttcgg atatggctaa      600
   accgcattta tcggtatagc gtgttaaccg gaccagattg ggaaagaaat gtgtcgagta      660
   acaaaaactg acatgcgctt ggcgcatccc agttggtaag aataaacggg actacttccg      720
   taatccggaa gagttttttt ccgaacaaat atg ttt gaa agg nnn atc gtg gct       774
                                    Met Phe Glu Arg Xaa Ile Val Ala
                                    1               5
   act gat aac aac aag gct gtc ctg cac tac ccc ggt ggc gag ttc gaa        822
   Thr Asp Asn Asn Lys Ala Val Leu His Tyr Pro Gly Gly Glu Phe Glu
       10                  15                  20
   atg gac atc atc gag gct tct gag ggt aac aac ggt gtt gtc ctg ggc        870
   Met Asp Ile Ile Glu Ala Ser Glu Gly Asn Asn Gly Val Val Leu Gly
   25                  30                  35                  40
   aag atg ctg tct gag act gga ctg atc act ttt gac cca ggt tat gtg        918
   Lys Met Leu Ser Glu Thr Gly Leu Ile Thr Phe Asp Pro Gly Tyr Val
                   45                  50                  55
   agc act ggc tcc acc gag tcg aag atc acc tac atc gat ggc gat gcg        966
   Ser Thr Gly Ser Thr Glu Ser Lys Ile Thr Tyr Ile Asp Gly Asp Ala
               60                  65                  70
   gga atc ctg cgt tac cgc ggc tat gac atc gct gat ctg gct gag aat       1014
   Gly Ile Leu Arg Tyr Arg Gly Tyr Asp Ile Ala Asp Leu Ala Glu Asn
           75                  80                  85
   gcc acc ttc aac gag gtt tct tac cta ctt atc aac ggt gag cta cca       1062
   Ala Thr Phe Asn Glu Val Ser Tyr Leu Leu Ile Asn Gly Glu Leu Pro
       90                  95                  100
   acc cca gat gag ctt cac aag ttt aac gac gag att cgc cac cac acc       1110
   Thr Pro Asp Glu Leu His Lys Phe Asn Asp Glu Ile Arg His His Thr
   105                 110                 115                 120
   ctt ctg gac gag gac ttc aag tcc cag ttc aac gtg ttc cca cgc gac       1158
   Leu Leu Asp Glu Asp Phe Lys Ser Gln Phe Asn Val Phe Pro Arg Asp
                   125                 130                 135
   gct cac cca atg gca acc ttg gct tcc tcg gtt aac att ttg tct acc       1206
   Ala His Pro Met Ala Thr Leu Ala Ser Ser Val Asn Ile Leu Ser Thr
               140                 145                 150
   tac tac cag gac cag ctg aac cca ctc gat gag gca cag ctt gat aag       1254
   Tyr Tyr Gln Asp Gln Leu Asn Pro Leu Asp Glu Ala Gln Leu Asp Lys
           155                 160                 165
   gca acc gtt cgc ctc atg gca aag gtt cca atg ctg gct gcg tac gca       1302
   Ala Thr Val Arg Leu Met Ala Lys Val Pro Met Leu Ala Ala Tyr Ala
       170                 175                 180
   cac cgc gca cgc aag ggt gct cct tac atg tac cca gac aac tcc ctc       1350
   His Arg Ala Arg Lys Gly Ala Pro Tyr Met Tyr Pro Asp Asn Ser Leu
   185                 190                 195                 200
   aat gcg cgt gag aac ttc ctg cgc atg atg ttc ggt tac cca acc gag       1398
   Asn Ala Arg Glu Asn Phe Leu Arg Met Met Phe Gly Tyr Pro Thr Glu
                   205                 210                 215
   cca tac gag atc gac cca atc atg gtc aag gct ctg gac aag ctg ctc       1446
   Pro Tyr Glu Ile Asp Pro Ile Met Val Lys Ala Leu Asp Lys Leu Leu
               220                 225                 230
   atc ctg cac gct gac cac gag cag aac tgc tcc acc tcc acc gtt cgt      1494
   Ile Leu His Ala Asp His Glu Gln Asn Cys Ser Thr Ser Thr Val Arg
           235                 240                 245
   atg atc ggt tcc gca cag gcc aac atg ttt gtc tcc atc gct ggt ggc      1542
   Met Ile Gly Ser Ala Gln Ala Asn Met Phe Val Ser Ile Ala Gly Gly
       250                 255                 260
   atc aac gct ctg tcc ggc cca ctg cac ggt ggc gca aac cag gct gtt      1590
   Ile Asn Ala Leu Ser Gly Pro Leu His Gly Gly Ala Asn Gln Ala Val
   265                 270                 275                 280
   ctg gag atg ctc gaa gac atc aag agc aac cac ggt ggc gac gca acc      1638
   Leu Glu Met Leu Glu Asp Ile Lys Ser Asn His Gly Gly Asp Ala Thr
                   285                 290                 295
   gag ttc atg aac aag gtc aag aac aag gaa gac ggc gtc cgc ctc atg      1686
   Glu Phe Met Asn Lys Val Lys Asn Lys Glu Asp Gly Val Arg Leu Met
               300                 305                 310
   ggc ttc gga cac cgc gtt tac aag aac tac gat cca cgt gca gca atc      1734
   Gly Phe Gly His Arg Val Tyr Lys Asn Tyr Asp Pro Arg Ala Ala Ile
           315                 320                 325
   gtc aag gag acc gca cac gag atc ctc gag cac ctc ggt ggc gac gat      1782
   Val Lys Glu Thr Ala His Glu Ile Leu Glu His Leu Gly Gly Asp Asp
       330                 335                 340
   ctt ctg gat ctg gca atc aag ctg gaa gaa att gca ctg gct gat gat      1830
   Leu Leu Asp Leu Ala Ile Lys Leu Glu Glu Ile Ala Leu Ala Asp Asp
   345                 350                 355                 360
   tac ttc atc tcc cgc aag ctc tac ccg aac gta gac ttc tac acc ggc      1878
   Tyr Phe Ile Ser Arg Lys Leu Tyr Pro Asn Val Asp Phe Tyr Thr Gly
                   365                 370                 375
   ctg atc tac cgc gca atg ggc ttc cca act gac ttc ttc acc gta ttg      1926
   Leu Ile Tyr Arg Ala Met Gly Phe Pro Thr Asp Phe Phe Thr Val Leu
               380                 385                 390
   ttc gca atc ggt cgt ctg cca gga tgg atc gct cac tac cgc gag cag      1974
   Phe Ala Ile Gly Arg Leu Pro Gly Trp Ile Ala His Tyr Arg Glu Gln
           395                 400                 405
   ctc ggt gca gca ggc aac aag atc aac cgc cca cgc cag gtc tac acc      2022
   Leu Gly Ala Ala Gly Asn Lys Ile Asn Arg Pro Arg Gln Val Tyr Thr
       410                 415                 420
   ggc aac gaa tcc cgc aag ttg gtt cct cgc gag gag cgc taaatttagc      2071
   Gly Asn Glu Ser Arg Lys Leu Val Pro Arg Glu Glu Arg
   425                 430                 435
   ggatgattct cgttcaactt cggccgaagc cacttcgtct gtcataatga cagggatggt      2131
   ttcggccgtt tttgcatgaa accaaaaaat acgattttca aggagcatgt acagcacatg      2191
   gaaaagccac agattgagct accggtcggt ccagcaccgg aagatctcgt aatctctgac      2251
   atcatcgttg gcgaaggagc agaagcccgc ccaggtggag aagttgaggt ccactatgtg      2311
   ggcgttgact ttgaaaccgg cgaggagttt gactcttcct gggatcgtgg acagaccagc      2371
   cagttcccac tcaacggcct cattgcaggt tggcaagagg gaattccagg catgaaggtc      2431
   ggcggacgtc gtcagctgac cattccgcca gaggctgctt acggccctga gggttccggc      2491
   cacccactgt ctggccgtac cctggtgttc atcatcgatt tgatcagcgc ataattttct 2551
   ttactgcgct aaacgctcaa atcgtgtgaa gcgactgtcg cgtcccgccc tctccggatt 2611
   gttatccaat tcggagaggg cgttgctgat tgtgccgaga atttcttcaa caaagtgctc 2671
   ggtttcggcg acgatcccgt cgataagccc ttggcttaaa agtgcgtgcg cctgcacgcc 2731
   ttgtcgctct atgatttccg cggcgtggtt ggtgtcgcgg aagaggatgg ccgaggcgcc 2791
   ctctggtggc aatgcggaca gcc 2814
<210> 10
   <211> 437
   <212> PRT
   <213> Corynebacterium glutamicum
<220>
   <221> misc_feature
   <222> (5)..(5)
   <223> The 'Xaa' at location 5 stands for Lys, Asn, Arg, Ser, Thr, Ile, Met, Glu, Asp, Gly, Ala, Val, Gln, His, Pro, Leu, Tyr, Trp, Cys, or Phe.
<400> 10
   Met Phe Glu Arg Xaa Ile Val Ala Thr Asp Asn Asn Lys Ala Val Leu
   1               5                   10                  15
   His Tyr Pro Gly Gly Glu Phe Glu Met Asp Ile Ile Glu Ala Ser Glu
               20                  25                  30
   Gly Asn Asn Gly Val Val Leu Gly Lys Met Leu Ser Glu Thr Gly Leu
           35                  40                  45
   Ile Thr Phe Asp Pro Gly Tyr Val Ser Thr Gly Ser Thr Glu Ser Lys
       50                  55                  60
   Ile Thr Tyr Ile Asp Gly Asp Ala Gly Ile Leu Arg Tyr Arg Gly Tyr
   65                  70                  75                  80
   Asp Ile Ala Asp Leu Ala Glu Asn Ala Thr Phe Asn Glu Val Ser Tyr
                   85                  90                  95
   Leu Leu Ile Asn Gly Glu Leu Pro Thr Pro Asp Glu Leu His Lys Phe
               100                 105                 110
   Asn Asp Glu Ile Arg His His Thr Leu Leu Asp Glu Asp Phe Lys Ser
           115                 120                 125
   Gln Phe Asn Val Phe Pro Arg Asp Ala His Pro Met Ala Thr Leu Ala
       130                 135                 140
   Ser Ser Val Asn Ile Leu Ser Thr Tyr Tyr Gln Asp Gln Leu Asn Pro
   145                 150                 155                 160
   Leu Asp Glu Ala Gln Leu Asp Lys Ala Thr Val Arg Leu Met Ala Lys
                   165                 170                 175
   Val Pro Met Leu Ala Ala Tyr Ala His Arg Ala Arg Lys Gly Ala Pro
   180 185 190
   Tyr Met Tyr Pro Asp Asn Ser Leu Asn Ala Arg Glu Asn Phe Leu Arg
   195 200 205
   Met Met Phe Gly Tyr Pro Thr Glu Pro Tyr Glu Ile Asp Pro Ile Met
   210 215 220
   Val Lys Ala Leu Asp Lys Leu Leu Ile Leu His Ala Asp His Glu Gln
   225 230 235 240
   Asn Cys Ser Thr Ser Thr Val Arg Met Ile Gly Ser Ala Gln Ala Asn
   245 250 255
   Met Phe Val Ser Ile Ala Gly Gly Ile Asn Ala Leu Ser Gly Pro Leu
   260 265 270
   His Gly Gly Ala Asn Gln Ala Val Leu Glu Met Leu Glu Asp Ile Lys
   275 280 285
   Ser Asn His Gly Gly Asp Ala Thr Glu Phe Met Asn Lys Val Lys Asn
   290 295 300
   Lys Glu Asp Gly Val Arg Leu Met Gly Phe Gly His Arg Val Tyr Lys
   305 310 315 320
   Asn Tyr Asp Pro Arg Ala Ala Ile Val Lys Glu Thr Ala His Glu Ile
   325 330 335
   Leu Glu His Leu Gly Gly Asp Asp Leu Leu Asp Leu Ala Ile Lys Leu
   340 345 350
   Glu Glu Ile Ala Leu Ala Asp Asp Tyr Phe Ile Ser Arg Lys Leu Tyr
   355 360 365
   Pro Asn Val Asp Phe Tyr Thr Gly Leu Ile Tyr Arg Ala Met Gly Phe
   370 375 380
   Pro Thr Asp Phe Phe Thr Val Leu Phe Ala Ile Gly Arg Leu Pro Gly
   385 390 395 400
   Trp Ile Ala His Tyr Arg Glu Gln Leu Gly Ala Ala Gly Asn Lys Ile
   405 410 415
   Asn Arg Pro Arg Gln Val Tyr Thr Gly Asn Glu Ser Arg Lys Leu Val
   420 425 430
   Pro Arg Glu Glu Arg
   435
<210> 11
   <211> 105
   <212> DNA
   <213> Corynebacterium glutamicum
<220>
   <221> CDS
   <222> (40)..(105)
<220>
   <221> misc_feature
   <222> (52)..(54)
   <223> n is a, c, g, or t
<400> 11
   ctacttccgt aatccggaag agtttttttc cgaacaaat atg ttt gaa agg nnn       54
                                              Met Phe Glu Arg Xaa
                                              1               5
   atc gtg gct act gat aac aac aag gct gtc ctg cac tac ccc ggt ggc      102
   Ile Val Ala Thr Asp Asn Asn Lys Ala Val Leu His Tyr Pro Gly Gly
                   10                  15                  20
   gag                                                                  105
   Glu
<210> 12
   <211> 22
   <212> PRT
   <213> Corynebacterium glutamicum
<220>
   <221> misc_feature
   <222> (5)..(5)
   <223> The 'Xaa' at location 5 stands for Lys, Asn, Arg, Ser, Thr, Ile, Met, Glu, Gly, Ala, Val, Gln, His, Pro, Leu, Tyr, Trp, Cys, or Phe.
<400> 12
   Met Phe Glu Arg Xaa Ile Val Ala Thr Asp Asn Asn Lys Ala Val Leu
   1               5                   10                  15
   His Tyr Pro Gly Gly Glu
               20
<210> 13
   <211> 206
   <212> DNA
   <213> Corynebacterium glutamicum
<220>
   <221> CDS
   <222> (90)..(206)
<220>
   <221> misc_feature
   <222> (102)..(104)
   <223> n is a, c, g, or t
<400> 13
   caaaaactga catgcgcttg gcgcatccca gttggtaaga ataaacggga ctacttccgt      60
   aatccggaag agtttttttc cgaacaaat atg ttt gaa agg nnn atc gtg gct        113
                                   Met Phe Glu Arg Xaa Ile Val Ala
                                   1               5
   act gat aac aac aag gct gtc ctg cac tac ccc ggt ggc gag ttc gaa        161
   Thr Asp Asn Asn Lys Ala Val Leu His Tyr Pro Gly Gly Glu Phe Glu
       10                  15                  20
   atg gac atc atc gag gct tct gag ggt aac aac ggt gtt gtc ctg            206
   Met Asp Ile Ile Glu Ala Ser Glu Gly Asn Asn Gly Val Val Leu
   25                  30                  35
<210> 14
   <211> 39
   <212> PRT
   <213> Corynebacterium glutamicum
<220>
   <221> misc_feature
   <222> (5)..(5)
   <223> The 'Xaa' at location 5 stands for Lys, Asn, Arg, Ser, Thr, Ile, Met, Glu, Gly, Ala, Val, Gln, His, Pro, Leu, Tyr, Trp, Cys, or Phe.
<400> 14
   Met Phe Glu Arg Xaa Ile Val Ala Thr Asp Asn Asn Lys Ala Val Leu
   1               5                   10                  15
   His Tyr Pro Gly Gly Glu Phe Glu Met Asp Ile Ile Glu Ala Ser Glu
               20                  25                  30
   Gly Asn Asn Gly Val Val Leu
           35
<210> 15
   <211> 405
   <212> DNA
   <213> Corynebacterium glutamicum
<220>
   <221> CDS
   <222> (190)..(405)
<220>
   <221> misc_feature
   <222> (202)..(204)
   <223> n is a, c, g, or t
<400> 15
   gattgtgggg gaattggctc tcacttcgga tatggctaaa ccgcatttat cggtatagcg      60
   tgttaaccgg accagattgg gaaagaaatg tgtcgagtaa caaaaactga catgcgcttg     120
   gcgcatccca gttggtaaga ataaacggga ctacttccgt aatccggaag agtttttttc     180
   cgaacaaat atg ttt gaa agg nnn atc gtg gct act gat aac aac aag gct    231
             Met Phe Glu Arg Xaa Ile Val Ala Thr Asp Asn Asn Lys Ala
             1               5                   10
   gtc ctg cac tac ccc ggt ggc gag ttc gaa atg gac atc atc gag gct       279
   Val Leu His Tyr Pro Gly Gly Glu Phe Glu Met Asp Ile Ile Glu Ala
   15                  20                  25                  30
   tct gag ggt aac aac ggt gtt gtc ctg ggc aag atg ctg tct gag act       327
   Ser Glu Gly Asn Asn Gly Val Val Leu Gly Lys Met Leu Ser Glu Thr
                   35                  40                  45
   gga ctg atc act ttt gac cca ggt tat gtg agc act ggc tcc acc gag       375
   Gly Leu Ile Thr Phe Asp Pro Gly Tyr Val Ser Thr Gly Ser Thr Glu
               50                  55                  60
   tcg aag atc acc tac atc gat ggc gat gcg                               405
   Ser Lys Ile Thr Tyr Ile Asp Gly Asp Ala
           65                  70
<210> 16
   <211> 72
   <212> PRT
   <213> Corynebacterium glutamicum
<220>
   <221> misc_feature
   <222> (5)..(5)
   <223> The 'Xaa' at location 5 stands for Lys, Asn, Arg, Ser, Thr, Ile, Met, Glu, Gly, Ala, Val, Gln, His, Pro, Leu, Tyr, Trp, Cys, or Phe.
<400> 16
   Met Phe Glu Arg Xaa Ile Val Ala Thr Asp Asn Asn Lys Ala Val Leu
   1               5                   10                  15
   His Tyr Pro Gly Gly Glu Phe Glu Met Asp Ile Ile Glu Ala Ser Glu
               20                  25                  30
   Gly Asn Asn Gly Val Val Leu Gly Lys Met Leu Ser Glu Thr Gly Leu
           35                  40                  45
   Ile Thr Phe Asp Pro Gly Tyr Val Ser Thr Gly Ser Thr Glu Ser Lys
       50                  55                  60
   Ile Thr Tyr Ile Asp Gly Asp Ala
   65                  70
<210> 17
   <211> 1001
   <212> DNA
   <213> Corynebacterium glutamicum
<220>
   <221> CDS
   <222> (489)..(1001)
<220>
   <221> misc_feature
   <222> (501)..(503)
   <223> n is a, c, g, or t
<400> 17
   gttgttaata tcccctgagg ttgcgttata gggtggcgaa ttgcatgggg aaagctactc      60
   ggcacccatc cttgtcgcgt gcatcacaaa ctttgctaaa ctgtgcacca gtccacttat     120
   tgtgggattt ttaatgcctt aaaggccagc attttcaccc tctagcgggg ttgaatgctg     180
   gccttgaggg tgcagaacta aatagcagca catcggcaca attgatctga gttctattgg     240
   cgtgaccgtg gctactgatt acggtggctg tgggtggtcg ggaatgatgt aaccaacgtg     300
   attgtggggg aattggctct cacttcggat atggctaaac cgcatttatc ggtatagcgt     360
   gttaaccgga ccagattggg aaagaaatgt gtcgagtaac aaaaactgac atgcgcttgg     420
   cgcatcccag ttggtaagaa taaacgggac tacttccgta atccggaaga gtttttttcc     480
   gaacaaat atg ttt gaa agg nnn atc gtg gct act gat aac aac aag gct      530
            Met Phe Glu Arg Xaa Ile Val Ala Thr Asp Asn Asn Lys Ala
            1               5                   10
   gtc ctg cac tac ccc ggt ggc gag ttc gaa atg gac atc atc gag gct       578
   Val Leu His Tyr Pro Gly Gly Glu Phe Glu Met Asp Ile Ile Glu Ala
   15                  20                  25                  30
   tct gag ggt aac aac ggt gtt gtc ctg ggc aag atg ctg tct gag act       626
   Ser Glu Gly Asn Asn Gly Val Val Leu Gly Lys Met Leu Ser Glu Thr
                   35                  40                  45
   gga ctg atc act ttt gac cca ggt tat gtg agc act ggc tcc acc gag       674
   Gly Leu Ile Thr Phe Asp Pro Gly Tyr Val Ser Thr Gly Ser Thr Glu
               50                  55                  60
   tcg aag atc acc tac atc gat ggc gat gcg gga atc ctg cgt tac cgc       722
   Ser Lys Ile Thr Tyr Ile Asp Gly Asp Ala Gly Ile Leu Arg Tyr Arg
           65                  70                  75
   ggc tat gac atc gct gat ctg gct gag aat gcc acc ttc aac gag gtt       770
   Gly Tyr Asp Ile Ala Asp Leu Ala Glu Asn Ala Thr Phe Asn Glu Val
       80                  85                  90
   tct tac cta ctt atc aac ggt gag cta cca acc cca gat gag ctt cac       818
   Ser Tyr Leu Leu Ile Asn Gly Glu Leu Pro Thr Pro Asp Glu Leu His
   95                  100                 105                 110
   aag ttt aac gac gag att cgc cac cac acc ctt ctg gac gag gac ttc       866
   Lys Phe Asn Asp Glu Ile Arg His His Thr Leu Leu Asp Glu Asp Phe
                   115                 120                 125
   aag tcc cag ttc aac gtg ttc cca cgc gac gct cac cca atg gca acc       914
   Lys Ser Gln Phe Asn Val Phe Pro Arg Asp Ala His Pro Met Ala Thr
               130                 135                 140
   ttg gct tcc tcg gtt aac att ttg tct acc tac tac cag gac cag ctg       962
   Leu Ala Ser Ser Val Asn Ile Leu Ser Thr Tyr Tyr Gln Asp Gln Leu
           145                 150                 155
   aac cca ctc gat gag gca cag ctt gat aag gca acc gtt                  1001
   Asn Pro Leu Asp Glu Ala Gln Leu Asp Lys Ala Thr Val
       160                 165                 170
<210> 18
   <211> 171
   <212> PRT
   <213> Corynebacterium glutamicum
<220>
   <221> misc_feature
   <222> (5)..(5)
   <223> The 'Xaa' at location 5 stands for Lys, Asn, Arg, Ser, Thr, Ile, Met, Glu, Gly, Ala, Val, Gln, His, Pro, Leu, Tyr, Trp, Cys, or Phe.
<400> 18
   Met Phe Glu Arg Xaa Ile Val Ala Thr Asp Asn Asn Lys Ala Val Leu
   1               5                   10                  15
   His Tyr Pro Gly Gly Glu Phe Glu Met Asp Ile Ile Glu Ala Ser Glu
               20                  25                  30
   Gly Asn Asn Gly Val Val Leu Gly Lys Met Leu Ser Glu Thr Gly Leu
           35                  40                  45
   Ile Thr Phe Asp Pro Gly Tyr Val Ser Thr Gly Ser Thr Glu Ser Lys
       50                  55                  60
   Ile Thr Tyr Ile Asp Gly Asp Ala Gly Ile Leu Arg Tyr Arg Gly Tyr
   65                  70                  75                  80
   Asp Ile Ala Asp Leu Ala Glu Asn Ala Thr Phe Asn Glu Val Ser Tyr
                   85                  90                  95
   Leu Leu Ile Asn Gly Glu Leu Pro Thr Pro Asp Glu Leu His Lys Phe
               100                 105                 110
   Asn Asp Glu Ile Arg His His Thr Leu Leu Asp Glu Asp Phe Lys Ser
           115                 120                 125
   Gln Phe Asn Val Phe Pro Arg Asp Ala His Pro Met Ala Thr Leu Ala
       130                 135                 140
   Ser Ser Val Asn Ile Leu Ser Thr Tyr Tyr Gln Asp Gln Leu Asn Pro
   145                 150                 155                 160
   Leu Asp Glu Ala Gln Leu Asp Lys Ala Thr Val
                   165                 170
<210> 19
   <211> 1263
   <212> DNA
   <213> Corynebacterium glutamicum
<220>
   <221> CDS
   <222> (1)..(1263)
   <223> lysC-wildtyp-Gen
<400> 19
   gtg gcc ctg gtc gta cag aaa tat ggc ggt tcc tcg ctt gag agt gcg      48
   Val Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct      96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat     144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt      192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc      240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg      288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc      336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc      384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc      432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg      480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt      528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag      576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct      1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg      1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt      1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca      1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat      1248
   Leu His Glu Gln Phe Gln Leu Gly Gly Glu Asp Glu Ala Val Val Tyr
                   405                 410                 415
   gca ggc acc gga cgc                                                  1263
   Ala Gly Thr Gly Arg
               420
<210> 20
   <211> 421
   <212> PRT
   <213> Corynebacterium glutamicum
<400> 20
   Val Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145 150 155 160
   Leu Ala Ala Ala Leu Asn Ala Asp Val Cys Glu Ile Tyr Ser Asp Val
   165 170 175
   Asp Gly Val Tyr Thr Ala Asp Pro Arg Ile Val Pro Asn Ala Gln Lys
   180 185 190
   Leu Glu Lys Leu Ser Phe Glu Glu Met Leu Glu Leu Ala Ala Val Gly
   195 200 205
   Ser Lys Ile Leu Val Leu Arg Ser Val Glu Tyr Ala Arg Ala Phe Asn
   210 215 220
   Val Pro Leu Arg Val Arg Ser Ser Tyr Ser Asn Asp Pro Gly Thr Leu
   225 230 235 240
   Ile Ala Gly Ser Met Glu Asp Ile Pro Val Glu Glu Ala Val Leu Thr
   245 250 255
   Gly Val Ala Thr Asp Lys Ser Glu Ala Lys Val Thr Val Leu Gly Ile
   260 265 270
   Ser Asp Lys Pro Gly Glu Ala Ala Lys Val Phe Arg Ala Leu Ala Asp
   275 280 285
   Ala Glu Ile Asn Ile Asp Met Val Leu Gln Asn Val Ser Ser Val Glu
   290 295 300
   Asp Gly Thr Thr Asp Ile Thr Phe Thr Cys Pro Arg Ser Asp Gly Arg
   305 310 315 320
   Arg Ala Met Glu Ile Leu Lys Lys Leu Gln Val Gln Gly Asn Trp Thr
   325 330 335
   Asn Val Leu Tyr Asp Asp Gln Val Gly Lys Val Ser Leu Val Gly Ala
   340 345 350
   Gly Met Lys Ser His Pro Gly Val Thr Ala Glu Phe Met Glu Ala Leu
   355 360 365
   Arg Asp Val Asn Val Asn Ile Glu Leu Ile Ser Thr Ser Glu Ile Arg
   370 375 380
   Ile Ser Val Leu Ile Arg Glu Asp Asp Leu Asp Ala Ala Ala Arg Ala
   385 390 395 400
   Leu His Glu Gln Phe Gln Leu Gly Gly Glu Asp Glu Ala Val Val Tyr
   405 410 415
   Ala Gly Thr Gly Arg
   420
<210> 21
   <211> 2266
   <212> DNA
   <213> Corynebacterium glutamicum
<220>
   <221> misc_feature
   <222> (1)..(500)
   <223> stromaufwärts der Kodierregion gelegene Sequenz
<220>
   <221> CDS
   <222> (501)..(1763)
   <223> Kodierregion des lysC-Wildtyp-Gens
<220>
   <221> misc_feature
   <222> (1764)..(2266)
   <223> stromabwärts der Kodierregion gelegene Nukleotidsequenz
<400> 21
   cgacaggaca agcactggtt gcactaccaa gagggtgccg aaaccaagtg ctactgtttg      60
   taagaaatat gccagcatcg cgtactcatg cctgcccacc acatcggtgt catcagagca     120
   ttgagtaaag gtgagctcct tagggagcca tcttttgggg tgcggagcgc gatccggtgt     180
   ctgaccacgg tgccccatgc gattgttaat gccgatgcta gggcgaaaag cacggcgagc     240
   agattgcttt gcacttgatt cagggtagtt gactaaagag ttgctcgcga agtagcacct     300
   gtcacttttg tctcaaatat taaatcgaat atcaatatat ggtctgttta ttggaacgcg     360
   tcccagtggc tgagacgcat ccgctaaagc cccaggaacc ctgtgcagaa agaaaacact     420
   cctctggcta ggtagacaca gtttataaag gtagagttga gcgggtaact gtcagcacgt     480
   agatcgaaag gtgcacaaag gtg gcc ctg gtc gta cag aaa tat ggc ggt tcc     533
                         Met Ala Leu Val Val Gln Lys Tyr Gly Gly Ser
                         1               5                   10
   tcg ctt gag agt gcg gaa cgc att aga aac gtc gct gaa cgg atc gtt       581
   Ser Leu Glu Ser Ala Glu Arg Ile Arg Asn Val Ala Glu Arg Ile Val
               15                  20                  25
   gcc acc aag aag gct gga aat gat gtc gtg gtt gtc tgc tcc gca atg       629
   Ala Thr Lys Lys Ala Gly Asn Asp Val Val Val Val Cys Ser Ala Met
           30                  35                  40
   gga gac acc acg gat gaa ctt cta gaa ctt gca gcg gca gtg aat ccc       677
   Gly Asp Thr Thr Asp Glu Leu Leu Glu Leu Ala Ala Ala Val Asn Pro
       45                  50                  55
   gtt ccg cca gct cgt gaa atg gat atg ctc ctg act gct ggt gag cgt       725
   Val Pro Pro Ala Arg Glu Met Asp Met Leu Leu Thr Ala Gly Glu Arg
   60                  65                  70                  75
   att tct aac gct ctc gtc gcc atg gct att gag tcc ctt ggc gca gaa       773
   Ile Ser Asn Ala Leu Val Ala Met Ala Ile Glu Ser Leu Gly Ala Glu
                   80                  85                  90
   gcc caa tct ttc acg ggc tct cag gct ggt gtg ctc acc acc gag cgc       821
   Ala Gln Ser Phe Thr Gly Ser Gln Ala Gly Val Leu Thr Thr Glu Arg
   95 100 105
   cac gga aac gca cgc att gtt gat gtc act cca ggt cgt gtg cgt gaa 869
   His Gly Asn Ala Arg Ile Val Asp Val Thr Pro Gly Arg Val Arg Glu
   110 115 120
   gca ctc gat gag ggc aag atc tgc att gtt gct ggt ttc cag ggt gtt 917
   Ala Leu Asp Glu Gly Lys Ile Cys Ile Val Ala Gly Phe Gln Gly Val
   125 130 135
   aat aaa gaa acc cgc gat gtc acc acg ttg ggt cgt ggt ggt tct gac 965
   Asn Lys Glu Thr Arg Asp Val Thr Thr Leu Gly Arg Gly Gly Ser Asp
   140 145 150 155
   acc act gca gtt gcg ttg gca gct gct ttg aac gct gat gtg tgt gag 1013
   Thr Thr Ala Val Ala Leu Ala Ala Ala Leu Asn Ala Asp Val Cys Glu
   160 165 170
   att tac tcg gac gtt gac ggt gtg tat acc gct gac ccg cgc atc gtt 1061
   Ile Tyr Ser Asp Val Asp Gly Val Tyr Thr Ala Asp Pro Arg Ile Val
   175 180 185
   cct aat gca cag aag ctg gaa aag ctc agc ttc gaa gaa atg ctg gaa 1109
   Pro Asn Ala Gln Lys Leu Glu Lys Leu Ser Phe Glu Glu Met Leu Glu
   190 195 200
   ctt gct gct gtt ggc tcc aag att ttg gtg ctg cgc agt gtt gaa tac 1157
   Leu Ala Ala Val Gly Ser Lys Ile Leu Val Leu Arg Ser Val Glu Tyr
   205 210 215
   gct cgt gca ttc aat gtg cca ctt cgc gta cgc tcg tct tat agt aat 1205
   Ala Arg Ala Phe Asn Val Pro Leu Arg Val Arg Ser Ser Tyr Ser Asn
   220 225 230 235
   gat ccc ggc act ttg att gcc ggc tct atg gag gat att cct gtg gaa 1253
   Asp Pro Gly Thr Leu Ile Ala Gly Ser Met Glu Asp Ile Pro Val Glu
   240 245 250
   gaa gca gtc ctt acc ggt gtc gca acc gac aag tcc gaa gcc aaa gta 1301
   Glu Ala Val Leu Thr Gly Val Ala Thr Asp Lys Ser Glu Ala Lys Val
   255 260 265
   acc gtt ctg ggt att tcc gat aag cca ggc gag gct gcg aag gtt ttc 1349
   Thr Val Leu Gly Ile Ser Asp Lys Pro Gly Glu Ala Ala Lys Val Phe
   270 275 280
   cgt gcg ttg gct gat gca gaa atc aac att gac atg gtt ctg cag aac 1397
   Arg Ala Leu Ala Asp Ala Glu Ile Asn Ile Asp Met Val Leu Gln Asn
   285 290 295
   gtc tct tct gta gaa gac ggc acc acc gac atc acc ttc acc tgc cct 1445
   Val Ser Ser Val Glu Asp Gly Thr Thr Asp Ile Thr Phe Thr Cys Pro
   300 305 310 315
   cgt tcc gac ggc cgc cgc gcg atg gag atc ttg aag aag ctt cag gtt 1493
   Arg Ser Asp Gly Arg Arg Ala Met Glu Ile Leu Lys Lys Leu Gln Val
   320 325 330
   cag ggc aac tgg acc aat gtg ctt tac gac gac cag gtc ggc aaa gtc 1541
   Gln Gly Asn Trp Thr Asn Val Leu Tyr Asp Asp Gln Val Gly Lys Val
   335 340 345
   tcc ctc gtg ggt gct ggc atg aag tct cac cca ggt gtt acc gca gag 1589
   Ser Leu Val Gly Ala Gly Met Lys Ser His Pro Gly Val Thr Ala Glu
   350 355 360
   ttc atg gaa gct ctg cgc gat gtc aac gtg aac atc gaa ttg att tcc 1637
   Phe Met Glu Ala Leu Arg Asp Val Asn Val Asn Ile Glu Leu Ile Ser
   365 370 375
   acc tct gag att cgt att tcc gtg ctg atc cgt gaa gat gat ctg gat      1685
   Thr Ser Glu Ile Arg Ile Ser Val Leu Ile Arg Glu Asp Asp Leu Asp
   380                 385                 390                 395
   gct gct gca cgt gca ttg cat gag cag ttc cag ctg ggc ggc gaa gac      1733
   Ala Ala Ala Arg Ala Leu His Glu Gln Phe Gln Leu Gly Gly Glu Asp
                   400                 405                 410
   gaa gcc gtc gtt tat gca ggc acc gga cgc taaagtttta aaggagtagt        1783
   Glu Ala Val Val Tyr Ala Gly Thr Gly Arg
               415                 420
   tttacaatga ccaccatcgc agttgttggt gcaaccggcc aggtcggcca ggttatgcgc    1843
   acccttttgg aagagcgcaa tttcccagct gacactgttc gtttctttgc ttccccacgt    1903
   tccgcaggcc gtaagattga attccgtggc acggaaatcg aggtagaaga cattactcag    1963
   gcaaccgagg agtccctcaa ggacatcgac gttgcgttgt tctccgctgg aggcaccgct    2023
   tccaagcagt acgctccact gttcgctgct gcaggcgcga ctgttgtgga taactcttct    2083
   gcttggcgca aggacgacga ggttccacta atcgtctctg aggtgaaccc ttccgacaag    2143
   gattccctgg tcaagggcat tattgcgaac cctaactgca ccaccatggc tgcgatgcca    2203
   gtgctgaagc cacttcacga tgccgctggt cttgtaaagc ttcacgtttc ctcttaccag    2263
   gct                                                                  2266
<210> 22
   <211> 421
   <212> PRT
   <213> Corynebacterium glutamicum
<400> 22
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
   130 135 140
   Asp Val Thr Thr Leu Gly Arg Gly Gly Ser Asp Thr Thr Ala Val Ala
   145 150 155 160
   Leu Ala Ala Ala Leu Asn Ala Asp Val Cys Glu Ile Tyr Ser Asp Val
   165 170 175
   Asp Gly Val Tyr Thr Ala Asp Pro Arg Ile Val Pro Asn Ala Gln Lys
   180 185 190
   Leu Glu Lys Leu Ser Phe Glu Glu Met Leu Glu Leu Ala Ala Val Gly
   195 200 205
   Ser Lys Ile Leu Val Leu Arg Ser Val Glu Tyr Ala Arg Ala Phe Asn
   210 215 220
   Val Pro Leu Arg Val Arg Ser Ser Tyr Ser Asn Asp Pro Gly Thr Leu
   225 230 235 240
   Ile Ala Gly Ser Met Glu Asp Ile Pro Val Glu Glu Ala Val Leu Thr
   245 250 255
   Gly Val Ala Thr Asp Lys Ser Glu Ala Lys Val Thr Val Leu Gly Ile
   260 265 270
   Ser Asp Lys Pro Gly Glu Ala Ala Lys Val Phe Arg Ala Leu Ala Asp
   275 280 285
   Ala Glu Ile Asn Ile Asp Met Val Leu Gln Asn Val Ser Ser Val Glu
   290 295 300
   Asp Gly Thr Thr Asp Ile Thr Phe Thr Cys Pro Arg Ser Asp Gly Arg
   305 310 315 320
   Arg Ala Met Glu Ile Leu Lys Lys Leu Gln Val Gln Gly Asn Trp Thr
   325 330 335
   Asn Val Leu Tyr Asp Asp Gln Val Gly Lys Val Ser Leu Val Gly Ala
   340 345 350
   Gly Met Lys Ser His Pro Gly Val Thr Ala Glu Phe Met Glu Ala Leu
   355 360 365
   Arg Asp Val Asn Val Asn Ile Glu Leu Ile Ser Thr Ser Glu Ile Arg
   370 375 380
   Ile Ser Val Leu Ile Arg Glu Asp Asp Leu Asp Ala Ala Ala Arg Ala
   385 390 395 400
   Leu His Glu Gln Phe Gln Leu Gly Gly Glu Asp Glu Ala Val Val Tyr
   405 410 415
   Ala Gly Thr Gly Arg
   420
<210> 23
   <211> 1503
   <212> DNA
   <213> Corynebacterium glutamicum
<220>
   <221> CDS
   <222> (1)..(1500)
   <223> mqo-Wildtyp-Gen
<400> 23
   atg tca gat tcc ccg aag aac gca ccg agg att acc gat gag gca gat 48
   Met Ser Asp Ser Pro Lys Asn Ala Pro Arg Ile Thr Asp Glu Ala Asp
   1 5 10 15
   gta gtt ctc att ggt gcc ggt atc atg agc tcc acg ctg ggt gca atg 96
   Val Val Leu Ile Gly Ala Gly Ile Met Ser Ser Thr Leu Gly Ala Met
   20 25 30
   ctg cgt cag ctg gag cca agc tgg act cag atc gtc ttc gag cgt ttg 144
   Leu Arg Gln Leu Glu Pro Ser Trp Thr Gln Ile Val Phe Glu Arg Leu
   35 40 45
   gat gga ccg gca caa gag tcg tcc tcc ccg tgg aac aat gca gga acc 192
   Asp Gly Pro Ala Gln Glu Ser Ser Ser Pro Trp Asn Asn Ala Gly Thr
   50 55 60
   ggc cac tct gct cta tgc gag ctg aac tac acc cca gag gtt aag ggc 240
   Gly His Ser Ala Leu Cys Glu Leu Asn Tyr Thr Pro Glu Val Lys Gly
   65 70 75 80
   aag gtt gaa att gcc aag gct gta gga atc aac gag aag ttc cag gtt 288
   Lys Val Glu Ile Ala Lys Ala Val Gly Ile Asn Glu Lys Phe Gln Val
   85 90 95
   tcc cgt cag ttc tgg tct cac ctc gtt gaa gag gga gtg ctg tct gat 336
   Ser Arg Gln Phe Trp Ser His Leu Val Glu Glu Gly Val Leu Ser Asp
   100 105 110
   cct aag gaa ttc atc aac cct gtt cct cac gta tct ttc ggc cag ggc 384
   Pro Lys Glu Phe Ile Asn Pro Val Pro His Val Ser Phe Gly Gln Gly
   115 120 125
   gca gat cag gtt gca tac atc aag gct cgc tac gaa gct ttg aag gat 432
   Ala Asp Gln Val Ala Tyr Ile Lys Ala Arg Tyr Glu Ala Leu Lys Asp
   130 135 140
   cac cca ctc ttc cag ggc atg acc tac gct gac gat gaa gct acc ttc 480
   His Pro Leu Phe Gln Gly Met Thr Tyr Ala Asp Asp Glu Ala Thr Phe
   145 150 155 160
   acc gag aag ctg cct ttg atg gca aag ggc cgt gac ttc tct gat cca 528
   Thr Glu Lys Leu Pro Leu Met Ala Lys Gly Arg Asp Phe Ser Asp Pro
   165 170 175
   gta gca atc tct tgg atc gat gaa ggc acc gac atc aac tac ggt gct 576
   Val Ala Ile Ser Trp Ile Asp Glu Gly Thr Asp Ile Asn Tyr Gly Ala
   180 185 190
   cag acc aag cag tac ctg gat gca gct gaa gtt gaa ggc act gaa atc        624
   Gln Thr Lys Gln Tyr Leu Asp Ala Ala Glu Val Glu Gly Thr Glu Ile
           195                 200                 205
   cgc tat ggc cac gaa gtc aag agc atc aag gct gat ggc gca aag tgg        672
   Arg Tyr Gly His Glu Val Lys Ser Ile Lys Ala Asp Gly Ala Lys Trp
       210                 215                 220
   atc gtg acc gtc aag aac gta cac act ggc gac acc aag acc atc aag        720
   Ile Val Thr Val Lys Asn Val His Thr Gly Asp Thr Lys Thr Ile Lys
   225                 230                 235                 240
   gca aac ttc gtg ttc gtc ggc gca ggc gga tac gca ctg gat ctg ctt        768
   Ala Asn Phe Val Phe Val Gly Ala Gly Gly Tyr Ala Leu Asp Leu Leu
                   245                 250                 255
   cgc agc gca ggc atc cca cag gtc aag ggc ttc gct gga ttc cca gta        816
   Arg Ser Ala Gly Ile Pro Gln Val Lys Gly Phe Ala Gly Phe Pro Val
               260                 265                 270
   tcc ggc ctg tgg ctt cgt tgc acc aac gag gaa ctg atc gag cag cac        864
   Ser Gly Leu Trp Leu Arg Cys Thr Asn Glu Glu Leu Ile Glu Gln His
           275                 280                 285
   gca gcc aag gta tat ggc aag gca tct gtt ggc gct cct cca atg tct        912
   Ala Ala Lys Val Tyr Gly Lys Ala Ser Val Gly Ala Pro Pro Met Ser
       290                 295                 300
   gtt cct cac ctt gac acc cgc gtt atc gag ggt gaa aag ggt ctg ctc        960
   Val Pro His Leu Asp Thr Arg Val Ile Glu Gly Glu Lys Gly Leu Leu
   305                 310                 315                 320
   ttt gga cct tac ggt ggc tgg acc cct aag ttc ttg aag gaa ggc tcc       1008
   Phe Gly Pro Tyr Gly Gly Trp Thr Pro Lys Phe Leu Lys Glu Gly Ser
                   325                 330                 335
   tac ctg gac ctg ttc aag tcc atc cgc cca gac aac att cct tcc tac       1056
   Tyr Leu Asp Leu Phe Lys Ser Ile Arg Pro Asp Asn Ile Pro Ser Tyr
               340                 345                 350
   ctt ggc gtt gct gct cag gaa ttt gat ctg acc aag tac ctt gtc act       1104
   Leu Gly Val Ala Ala Gln Glu Phe Asp Leu Thr Lys Tyr Leu Val Thr
           355                 360                 365
   gaa gtt ctc aag gac cag gac aag cgt atg gat gct ctt cgc gag tac       1152
   Glu Val Leu Lys Asp Gln Asp Lys Arg Met Asp Ala Leu Arg Glu Tyr
       370                 375                 380
   atg cca gag gca caa aac ggc gat tgg gag acc atc gtt gcc gga cag       1200
   Met Pro Glu Ala Gln Asn Gly Asp Trp Glu Thr Ile Val Ala Gly Gln
   385                 390                 395                 400
   cgt gtt cag gtt att aag cct gca gga ttc cct aag ttc ggt tcc ctg       1248
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga       1296
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc       1344
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac       1392
   Glu Leu Leu Glu Arg Cys Phe Gly Asp Arg Met Ile Glu Trp Gly Asp
       450                 455                 460
   aag ctg aag gac atg atc cct tcc tac ggc aag aag ctt gct tcc gag       1440
   Lys Leu Lys Asp Met Ile Pro Ser Tyr Gly Lys Lys Leu Ala Ser Glu
   465                 470                 475                 480
   cca gca ctg ttt gag cag cag tgg gca cgc acc cag aag acc ctg aag      1488
   Pro Ala Leu Phe Glu Gln Gln Trp Ala Arg Thr Gln Lys Thr Leu Lys
                   485                 490                 495
   ctt gag gaa gcc taa                                                  1503
   Leu Glu Glu Ala
               500
<210> 24
   <211> 500
   <212> PRT
   <213> Corynebacterium glutamicum
<400> 24
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ser Asp
               100                 105                 110
   Pro Lys Glu Phe Ile Asn Pro Val Pro His Val Ser Phe Gly Gln Gly
           115                 120                 125
   Ala Asp Gln Val Ala Tyr Ile Lys Ala Arg Tyr Glu Ala Leu Lys Asp
       130                 135                 140
   His Pro Leu Phe Gln Gly Met Thr Tyr Ala Asp Asp Glu Ala Thr Phe
   145                 150                 155                 160
   Thr Glu Lys Leu Pro Leu Met Ala Lys Gly Arg Asp Phe Ser Asp Pro
                   165                 170                 175
   Val Ala Ile Ser Trp Ile Asp Glu Gly Thr Asp Ile Asn Tyr Gly Ala
               180                 185                 190
   Gln Thr Lys Gln Tyr Leu Asp Ala Ala Glu Val Glu Gly Thr Glu Ile
           195                 200                 205
   Arg Tyr Gly His Glu Val Lys Ser Ile Lys Ala Asp Gly Ala Lys Trp
       210                 215                 220
   Ile Val Thr Val Lys Asn Val His Thr Gly Asp Thr Lys Thr Ile Lys
   225                 230                 235                 240
   Ala Asn Phe Val Phe Val Gly Ala Gly Gly Tyr Ala Leu Asp Leu Leu
                   245                 250                 255
   Arg Ser Ala Gly Ile Pro Gln Val Lys Gly Phe Ala Gly Phe Pro Val
               260                 265                 270
   Ser Gly Leu Trp Leu Arg Cys Thr Asn Glu Glu Leu Ile Glu Gln His
           275                 280                 285
   Ala Ala Lys Val Tyr Gly Lys Ala Ser Val Gly Ala Pro Pro Met Ser
       290                 295                 300
   Val Pro His Leu Asp Thr Arg Val Ile Glu Gly Glu Lys Gly Leu Leu
   305                 310                 315                 320
   Phe Gly Pro Tyr Gly Gly Trp Thr Pro Lys Phe Leu Lys Glu Gly Ser
                   325                 330                 335
   Tyr Leu Asp Leu Phe Lys Ser Ile Arg Pro Asp Asn Ile Pro Ser Tyr
               340                 345                 350
   Leu Gly Val Ala Ala Gln Glu Phe Asp Leu Thr Lys Tyr Leu Val Thr
           355                 360                 365
   Glu Val Leu Lys Asp Gln Asp Lys Arg Met Asp Ala Leu Arg Glu Tyr
       370                 375                 380
   Met Pro Glu Ala Gln Asn Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr Leu Lys
   485 490 495
   Leu Glu Glu Ala
   500
<210> 25
   <211> 3314
   <212> DNA
   <213> Corynebacterium glutamicum
<220>
   <221> misc_feature
   <222> (1)..(1000)
   <223> stromaufwärts der Kodierregion gelegene Sequenz
<220>
   <221> CDS
   <222> (1001)..(2311)
   <223> gltA-Wildtyp-Gen
<220>
   <221> misc_feature
   <222> (1013)..(1015)
<220>
   <221> misc_feature
   <222> (2312)..(3314)
   <223> stromabwärts der Kodierregion gelegene Sequenz
<400> 25
   atgagtccga aggttgctgc atcccagaat gcggtcgcac cacctaggga aaggatgatc     60
   tcgtagcctt ctggaaggga gaagaggtcg gagagtccct cgcggattga acccacgacg    120
   ttttttactg ccggctgacg gtgtgaggta ccgatgacgg atgcggatcc gtcgacaata     180
   gcctgaatct gttctggtcg aaccttggaa ggtccgcagc cgaaacggcc gtcgccaggg    240
   atgaactcag agggcagggt ggggaagtcg gtcatgtctt cgggcaactt tctgcgcttg    300
   gaagtaaaag ggccagggat cgttaacgat ctgacccaac aactataacc ctgaagctgt    360
   cagttcctag caccctagat tcttcacgca gtctcccaaa cgatgaaaaa cgcccaaaac    420
   tggcgacacc gaactattga aaacgcgggg attagttgac cagccaccaa tttgggggta    480
   gctcaaagtt ttgcaaagtt ttcaatttct aggttgttaa tatcccctga ggttgcgtta    540
   tagggtggcg aattgcatgg ggaaagctac tcggcaccca tccttgtcgc gtgcatcaca    600
   aactttgcta aactgtgcac cagtccactt attgtgggat ttttaatgcc ttaaaggcca    660
   gcattttcac cctctagcgg ggttgaatgc tggccttgag ggtgcagaac taaatagcag    720
   cacatcggca caattgatct gagttctatt ggcgtgaccg tggctactga ttacggtggc    780
   tgtgggtggt cgggaatgat gtaaccaacg tgattgtggg ggaattggct ctcacttcgg    840
   atatggctaa accgcattta tcggtatagc gtgttaaccg gaccagattg ggaaagaaat    900
   gtgtcgagta acaaaaactg acatgcgctt ggcgcatccc agttggtaag aataaacggg    960
   actacttccg taatccggaa gagttttttt ccgaacaaat atg ttt gaa agg gat     1015
   atc gtg gct act gat aac aac aag gct gtc ctg cac tac ccc ggt ggc      1063
   gag ttc gaa atg gac atc atc gag gct tct gag ggt aac aac ggt gtt      1111
   gtc ctg ggc aag atg ctg tct gag act gga ctg atc act ttt gac cca      1159
   ggt tat gtg agc act ggc tcc acc gag tcg aag atc acc tac atc gat      1207
   ggc gat gcg gga atc ctg cgt tac cgc ggc tat gac atc gct gat ctg      1255
   gct gag aat gcc acc ttc aac gag gtt tct tac cta ctt atc aac ggt      1303
   gag cta cca acc cca gat gag ctt cac aag ttt aac gac gag att cgc      1351
   cac cac acc ctt ctg gac gag gac ttc aag tcc cag ttc aac gtg ttc      1399
   cca cgc gac gct cac cca atg gca acc ttg gct tcc tcg gtt aac att      1447
   ttg tct acc tac tac cag gac cag ctg aac cca ctc gat gag gca cag      1495
   ctt gat aag gca acc gtt cgc ctc atg gca aag gtt cca atg ctg gct      1543
   gcg tac gca cac cgc gca cgc aag ggt gct cct tac atg tac cca gac      1591
   aac tcc ctc aat gcg cgt gag aac ttc ctg cgc atg atg ttc ggt tac      1639
   cca acc gag cca tac gag atc gac cca atc atg gtc aag gct ctg gac      1687
   aag ctg ctc atc ctg cac gct gac cac gag cag aac tgc tcc acc tcc      1735
   acc gtt cgt atg atc ggt tcc gca cag gcc aac atg ttt gtc tcc atc      1783
   gct ggt ggc atc aac gct ctg tcc ggc cca ctg cac ggt ggc gca aac      1831
   cag gct gtt ctg gag atg ctc gaa gac atc aag agc aac cac ggt ggc     1879
   gac gca acc gag ttc atg aac aag gtc aag aac aag gaa gac ggc gtc     1927
   cgc ctc atg ggc ttc gga cac cgc gtt tac aag aac tac gat cca cgt     1975
   gca gca atc gtc aag gag acc gca cac gag atc ctc gag cac ctc ggt     2023
   ggc gac gat ctt ctg gat ctg gca atc aag ctg gaa gaa att gca ctg     2071
   gct gat gat tac ttc atc tcc cgc aag ctc tac ccg aac gta gac ttc     2119
   tac acc ggc ctg atc tac cgc gca atg ggc ttc cca act gac ttc ttc     2167
   acc gta ttg ttc gca atc ggt cgt ctg cca gga tgg atc gct cac tac     2215
   cgc gag cag ctc ggt gca gca ggc aac aag atc aac cgc cca cgc cag     2263
   gtc tac acc ggc aac gaa tcc cgc aag ttg gtt cct cgc gag gag cgc     2311
   taaatttagc ggatgattct cgttcaactt cggccgaagc cacttcgtct gtcataatga   2371
   cagggatggt ttcggccgtt tttgcatgaa accaaaaaat acgattttca aggagcatgt   2431
   acagcacatg gaaaagccac agattgagct accggtcggt ccagcaccgg aagatctcgt   2491
   aatctctgac atcatcgttg gcgaaggagc agaagcccgc ccaggtggag aagttgaggt   2551
   ccactatgtg ggcgttgact ttgaaaccgg cgaggagttt gactcttcct gggatcgtgg   2611
   acagaccagc cagttcccac tcaacggcct cattgcaggt tggcaagagg gaattccagg   2671
   catgaaggtc ggcggacgtc gtcagctgac cattccgcca gaggctgctt acggccctga   2731
   gggttccggc cacccactgt ctggccgtac cctggtgttc atcatcgatt tgatcagcgc   2791
   ataattttct ttactgcgct aaacgctcaa atcgtgtgaa gcgactgtcg cgtcccgccc   2851
   tctccggatt gttatccaat tcggagaggg cgttgctgat tgtgccgaga atttcttcaa   2911
   caaagtgctc ggtttcggcg acgatcccgt cgataagccc ttggcttaaa agtgcgtgcg   2971
   cctgcacgcc ttgtcgctct atgatttccg cggcgtggtt ggtgtcgcgg aagaggatgg   3031
   ccgaggcgcc ctctggtggc aatgcggaca gccacgcgtt ttcggccgcg tagaccagat   3091
   cggcgggcag catggccagc gcgccaccgc caacgccctg accaataatg accgaaacgg   3151
   tggggagggg agcgtcgata agcttggaca aggtgcgcgc aatcgagctt gcgatgccga 3211
   gctcctcagc cgcctgcgac aattcggcgc cggaggtgtc gatgatggac acgatcggca 3271
   ggtttagctc gcgcgccagc gaaatgccac gacgcgcaaa acg 3314
<210> 26
   <211> 437
   <212> PRT
   <213> Corynebacterium glutamicum
<400> 26
<210> 27
   <211> 20
   <212> DNA
   <213> Artificial Sequence
<220>
   <223> Primer gltA-XL-A1
<400> 27
   tgagttctat tggcgtgacc 20
<210> 28
   <211> 20
   <212> DNA
   <213> Artificial Sequence
<220>
   <223> Primer gltA-XL-E1
<400> 28
   ttcgccaacg atgatgtcag 20
<210> 29
   <211> 19
   <212> DNA
   <213> Artificial Sequence
<220>
   <223> Primer gltA_1.p
<400> 29
   ccgtcgacaa tagcctgaa 19
<210> 30
   <211> 26
   <212> DNA
   <213> Artificial Sequence
<220>
   <223> Primer gltA_2.p
<400> 30
   ccgaattctt cgagcatctc cagaac 26
<210> 31
   <211> 1695
   <212> DNA
   <213> Corynebacterium glutamicum
<220>
   <221> misc_feature
   <222> (1)..(2)
   <223> Sequenz CC
<220>
   <221> misc_feature
   <222> (3)..(8)
   <223> SalI Restriktionsschnittstelle
<220>
   <221> misc_feature
   <222> (9)..(832)
   <223> stromaufwärts der Kodierregion gelegene Sequenz
<220>
   <221> CDS
   <222> (833)..(1687)
   <223> N terminaler Teil der Kodierregion,
<220>
   <221> mutation
   <222> (847)..(847)
   <223> Transversion A > T
<220>
   <221> misc_feature
   <222> (1688)..(1693)
   <223> EcoRI Restriktionsschnittstelle
<220>
   <221> misc_feature
   <222> (1694)..(1695)
   <223> Sequenz GG
<400> 31
   ccgtcgacaa tagcctgaat ctgttctggt cgaaccttgg aaggtccgca gccgaaacgg      60
   ccgtcgccag ggatgaactc agagggcagg gtggggaagt cggtcatgtc ttcgggcaac     120
   tttctgcgct tggaagtaaa agggccaggg atcgttaacg atctgaccca acaactataa     180
   ccctgaagct gtcagttcct agcaccctag attcttcacg cagtctccca aacgatgaaa     240
   aacgcccaaa actggcgaca ccgaactatt gaaaacgcgg ggattagttg accagccacc     300
   aatttggggg tagctcaaag ttttgcaaag ttttcaattt ctaggttgtt aatatcccct     360
   gaggttgcgt tatagggtgg cgaattgcat ggggaaagct actcggcacc catccttgtc     420
   gcgtgcatca caaactttgc taaactgtgc accagtccac ttattgtggg atttttaatg     480
   ccttaaaggc cagcattttc accctctagc ggggttgaat gctggccttg agggtgcaga     540
   actaaatagc agcacatcgg cacaattgat ctgagttcta ttggcgtgac cgtggctact     600
   gattacggtg gctgtgggtg gtcgggaatg atgtaaccaa cgtgattgtg ggggaattgg     660
   ctctcacttc ggatatggct aaaccgcatt tatcggtata gcgtgttaac cggaccagat     720
   tgggaaagaa atgtgtcgag taacaaaaac tgacatgcgc ttggcgcatc ccagttggta     780
   agaataaacg ggactacttc cgtaatccgg aagagttttt ttccgaacaa at atg ttt     838
                                                             Met Phe
                                                             1
   gaa agg gtt atc gtg gct act gat aac aac aag gct gtc ctg cac tac        886
   Glu Arg Val Ile Val Ala Thr Asp Asn Asn Lys Ala Val Leu His Tyr
           5                   10                  15
   ccc ggt ggc gag ttc gaa atg gac atc atc gag gct tct gag ggt aac        934
   Pro Gly Gly Glu Phe Glu Met Asp Ile Ile Glu Ala Ser Glu Gly Asn
       20                  25                  30
   aac ggt gtt gtc ctg ggc aag atg ctg tct gag act gga ctg atc act        982
   Asn Gly Val Val Leu Gly Lys Met Leu Ser Glu Thr Gly Leu Ile Thr
   35                  40                  45                  50
   ttt gac cca ggt tat gtg agc act ggc tcc acc gag tcg aag atc acc       1030
   Phe Asp Pro Gly Tyr Val Ser Thr Gly Ser Thr Glu Ser Lys Ile Thr
                   55                  60                  65
   tac atc gat ggc gat gcg gga atc ctg cgt tac cgc ggc tat gac atc       1078
   Tyr Ile Asp Gly Asp Ala Gly Ile Leu Arg Tyr Arg Gly Tyr Asp Ile
               70                  75                  80
   gct gat ctg gct gag aat gcc acc ttc aac gag gtt tct tac cta ctt       1126
   Ala Asp Leu Ala Glu Asn Ala Thr Phe Asn Glu Val Ser Tyr Leu Leu
           85                  90                  95
   atc aac ggt gag cta cca acc cca gat gag ctt cac aag ttt aac gac       1174
   Ile Asn Gly Glu Leu Pro Thr Pro Asp Glu Leu His Lys Phe Asn Asp
       100                 105                 110
   gag att cgc cac cac acc ctt ctg gac gag gac ttc aag tcc cag ttc       1222
   Glu Ile Arg His His Thr Leu Leu Asp Glu Asp Phe Lys Ser Gln Phe
   115                 120                 125                 130
   aac gtg ttc cca cgc gac gct cac cca atg gca acc ttg gct tcc tcg      1270
   Asn Val Phe Pro Arg Asp Ala His Pro Met Ala Thr Leu Ala Ser Ser
                   135                 140                 145
   gtt aac att ttg tct acc tac tac cag gac cag ctg aac cca ctc gat      1318
   Val Asn Ile Leu Ser Thr Tyr Tyr Gln Asp Gln Leu Asn Pro Leu Asp
               150                 155                 160
   gag gca cag ctt gat aag gca acc gtt cgc ctc atg gca aag gtt cca      1366
   Glu Ala Gln Leu Asp Lys Ala Thr Val Arg Leu Met Ala Lys Val Pro
           165                 170                 175
   atg ctg gct gcg tac gca cac cgc gca cgc aag ggt gct cct tac atg      1414
   Met Leu Ala Ala Tyr Ala His Arg Ala Arg Lys Gly Ala Pro Tyr Met
       180                 185                 190
   tac cca gac aac tcc ctc aat gcg cgt gag aac ttc ctg cgc atg atg      1462
   Tyr Pro Asp Asn Ser Leu Asn Ala Arg Glu Asn Phe Leu Arg Met Met
   195                 200                 205                 210
   ttc ggt tac cca acc gag cca tac gag atc gac cca atc atg gtc aag      1510
   Phe Gly Tyr Pro Thr Glu Pro Tyr Glu Ile Asp Pro Ile Met Val Lys
                   215                 220                 225
   gct ctg gac aag ctg ctc atc ctg cac gct gac cac gag cag aac tgc      1558
   Ala Leu Asp Lys Leu Leu Ile Leu His Ala Asp His Glu Gln Asn Cys
               230                 235                 240
   tcc acc tcc acc gtt cgt atg atc ggt tcc gca cag gcc aac atg ttt      1606
   Ser Thr Ser Thr Val Arg Met Ile Gly Ser Ala Gln Ala Asn Met Phe
           245                 250                 255
   gtc tcc atc gct ggt ggc atc aac gct ctg tcc ggc cca ctg cac ggt      1654
   Val Ser Ile Ala Gly Gly Ile Asn Ala Leu Ser Gly Pro Leu His Gly
       260                 265                 270
   ggc gca aac cag gct gtt ctg gag atg ctc gaa gaattcgg                 1695
   Gly Ala Asn Gln Ala Val Leu Glu Met Leu Glu
   275                 280                 285